# EUROPÄISCHE PATENTSCHRIFT

(11) **EP 3 984 482 B1**
(45) Veröffentlichungstag und Bekanntmachung des Hinweises auf die Patenterteilung: **23.04.2025**
(21) Anmeldenummer: 20202484.0
(22) Anmeldetag: 19.10.2020
(51) Int. Cl.: A61B 18/14, A61B 18/00, A61B 5/00

(54) **VORRICHTUNG ZUR BEFESTIGUNG EINER LICHTAUFNAHMEEINRICHTUNG AN EINEM CHIRURGISCHEN INSTRUMENT**
DEVICE FOR FIXING A LIGHT HOLDING DEVICE TO A SURGICAL INSTRUMENT
DISPOSITIF DE FIXATION D'UN DISPOSITIF DE RÉCEPTION DE LUMIÈRE AU NIVEAU D'UN INSTRUMENT CHIRURGICAL

(43) Veröffentlichungstag der Anmeldung: 20.04.2022
(73) Patentinhaber: Erbe Elektromedizin GmbH, 72072 Tübingen (DE)
(72) Erfinder: Reiterer, Markus, 2620 Loipersbach (AT); Fischer, Klaus, 72202 Nagold (DE); Brodbeck, Achim, 72555 Metzingen (DE); Müller, Marc, 72074 Tübingen (DE)
(74) Vertreter: Rüger Abel Patentanwälte PartGmbB

(56) Entgegenhaltungen:
- DE-A1- 19 735 150
- US-A1- 2012 289 954
- US-A1- 2018 078 301

## Beschreibung

Die vorliegende Offenbarung betrifft das Gebiet der Analyse von Lichterscheinungen, z.B. Funken, welche HFchirurgische Instrumente hervorrufen können.

US 2018/0078301 A1 beschreibt ein elektrochirurgisches Instrument mit einer Beleuchtung. Dadurch soll die Verwendung des elektrochirurgischen Instruments in dunklen Öffnungen verbessert werden.

Aus DE 197 35 150 A1 ist eine Zusatzvorrichtung für ein Endoskop bekannt, um Fluoreszenzlicht und/oder remittiertes Licht zu erfassen. Im Endoskoprohr ist ein bildgebender Kanal sowie ein Beleuchtungskanal zusätzlich zum Arbeitskanal vorhanden. Eine optische Zusatzvorrichtung leitet emittiertes Fluoreszenzlicht und/oder remittiertes Licht zu einem Spektrometer. An das Spektrometer kann ein Monitor zur Darstellung der Spektren angeschlossen sein.

In den Anmeldungen EP 3 284 430 A1 und EP 2 659 846 A1 ist ein Instrument mit einer Elektrode und Lichtaufnahmeeinrichtungen offenbart, die mit unterschiedlicher Ausrichtung auf die Elektrode schauend ausgerichtet sein können.

US 2009/0088772 A1 zeigt ein von einem Roboter tragbares Instrument, welches einen distalen Arbeitsabschnitt und eine optische Faser aufweist, welche Licht zur Analyse des Lichtes von dem distalen Ende des Instruments übertragen kann.

In EP 2 815 713 A1 ist ein Lichtleiter in einer Elektrode angeordnet.

Um das Licht einer Lichterscheinung, insbesondere eines HF-Funkens, hervorgerufen durch einen HFchirurgischen Eingriff im klinischen Einsatz zu analysieren, werden als Lichtaufnahmeeinrichtung optische Fasern eingesetzt, die mit einer Analyseeinrichtung zur Bestimmung von Lichtmerkmalen verbunden ist und dem Anwender während dem Eingriff Information zum biologischen Gewebe wiedergeben kann. Durch Blut, Gewebeflüssigkeit, Rauchgas (Aerosol), durch Gewebekontakt des distalen Endes der optischen Faser sowie Kontakt der optischen Faser zum Gewebe, durch Abplatzen des Gewebes und Wegspritzen von Gewebepartikeln kann die optische Faser verschmutzen und so die Transmission des Lichtes zur Ausführeinheit merklich reduzieren oder sogar ganz verhindern. Auch durch Anhaften von Gewebe am distalen Ende der Analyseeinrichtung, was indirekt zur Abschattung der optischen Faser führen kann, ist eine Reduktion der Transmission des Lichtes möglich. Eine Verschmutzung der optischen Faser kann das Lichtsignal in seiner Intensität verfälschen, da das Licht von der Verschmutzung unterschiedlich stark absorbiert werden kann. Auch eine Kondensation von Flüssigkeit auf der Faser kann die Transmission kurzzeitig verändern. Durch Verschmutzung kann die Transmission auch wellenlängenabhängig verändert werden, was zu einer Verfälschung des Analyseergebnisses führen kann. Durch einen hohen Energieeintrag (zum Beispiel 300 Watt) bei der HF-Chirurgie können sehr hohe Temperaturen (zum Beispiel größer 300°C) an der Spitze der HF-Elektrode entstehen. Auch hohe Temperaturen können die optische Faser thermisch schädigen, wodurch ebenfalls die Transmission des Lichtes reduziert oder ganz verhindert werden kann.

Aufgabe der vorliegenden Erfindung ist es, ein verbessertes Konzept für eine Lichtaufnahmeeinrichtung anzugeben.

Diese Aufgabe wird mit einer Vorrichtung gemäß Anspruch 1 und mit einem Verfahren gemäß Anspruch 15 gelöst**.**

Die erfindungsgemäße Vorrichtung ist dazu eingerichtet, bevorzugt lösbar und insbesondere zerstörungsfrei lösbar an einem chirurgischen Instrument, insbesondere einem elektrochirurgischen Instrument, oder an einer Instrumentenkomponente des chirurgischen Instruments befestigt zu werden. Die Vorrichtung weist eine Lichtaufnahmeeinrichtung (insbesondere optische Faser) zur Lichtanalyse auf, insbesondere durch einen medizinisch geschulten Anwender, beispielsweise einen Arzt. Die Befestigung kann während einer Operation (im Operationssaal) erfolgen, beispielsweise nachdem mit dem Instrument ein Operationsschritt durchgeführt wurde. Die Vorrichtung ermöglicht mittels der Lichtaufnahmeeinrichtung (insbesondere optische Faser) das Aufnehmen bzw. Empfangen von Licht, das bei der Verwendung des chirurgischen Instruments erzeugt wird. Das empfangene Licht kann mittels der Lichtaufnahmeeinrichtung zur Lichtanalyse weitergeleitet werden. .Die Lichtaufnahmeeinheit der Vorrichtung kann also bei der Verwendung des Instruments für die Lichtanalyse geeignet Licht aufnehmen.

Mittels der Vorrichtung kann die Lichtaufnahmeeinrichtung beispielsweise nur dann an einem Handgriff des Instruments befestigt werden, wenn die Gewinnung eine Gewebeinformation durch Lichtanalyse klinisch gewünscht ist. Mit bevorzugten Ausführungsformen zur lösbaren Befestigung kann die Lichtaufnahmeeinrichtung von dem Anwender leicht durch eine neue, saubere Lichtaufnahmeeinrichtung ausgetauscht werden. Die Vorrichtung muss dafür bevorzugt nicht zerstört werden. Die Verbindung der Lichtaufnahmeeinrichtung mit dem chirurgischen Instrument mittels der Vorrichtung ist bevorzugt werkzeuglos möglich. Die Vorrichtung kann vorzugsweise auf das Instrument gesteckt oder, zumindest abschnittsweise, in das Instrument geschoben werden und/oder das Instrument kann vorzugsweise in die Vorrichtung gesteckt oder, zumindest abschnittsweise, in die Vorrichtung geschoben werden und/oder die Vorrichtung kann in das Instrument gesteckt oder, zumindest abschnittsweise, geschoben werden.

Erfindungsgemäß wird ein Verfahren offenbart, welches das Befestigen einer Lichtaufnahmeeinrichtung an einem chirurgischen Instrument oder einer Instrumentenkomponente des chirurgischen Instruments durch den Chirurgen oder seinen Assistenten während einer Operation aufweist. Der Chirurg oder sein Assistent kombiniert die Lichtaufnahmeeinrichtung mit dem Instrument oder der Instrumentenkomponente zu einer arbeitsfähigen Einheit, mittels welcher in Ausführungsformen insbesondere ein HF-chirurgischer Eingriff unter Überwachung mittels optischer Emissionsspektroskopie durchgeführt werden kann. Der Chirurg oder sein Assistent kann dazu beispielsweise eine erfindungsgemäße Vorrichtung wie hierin beschrieben verwenden.

Die erfindungsgemäße Vorrichtung kann beispielsweise durch wenigstens eines oder mehrere der nachfolgend angegebenen Merkmale weitergebildet werden.

Die Instrumentenkomponente des chirurgischen Instruments kann beispielsweise ein Bauteil oder eine Baugruppe des Instruments sein.

Bevorzugt ist die Vorrichtung zur Befestigung, während einer Operation (im Operationssaal), beispielsweise nachdem mit dem Instrument ein Operationsschritt durchgeführt wurde, einer Lichtaufnahmeeinrichtung (insbesondere optische Faser) zur Lichtanalyse an einem arbeitsfähigen chirurgischen Instrument durch einen medizinisch geschulten Anwender, beispielsweise einen Arzt eingerichtet. Das Instrument ist in Ausführungsformen also bereits ohne die Vorrichtung und die Lichtaufnahmeeinrichtung arbeitsfähig, wenn die Lichtaufnahmeeinrichtung mittels der Vorrichtung an dem Instrument befestigt wird. Die Instrumentenkomponente kann beispielsweise bereits vor dem Verbinden mit der Vorrichtung mit einer Elektrode verbunden sein, so dass die Instrumentenkomponente mit der Elektrode ein arbeitsfähiges HF-chirurgisches Instrument bildet.

Alternativ oder zusätzlich ist die Vorrichtung bevorzugt dazu eingerichtet, die Instrumentenkomponente beim Befestigen der Lichtaufnahmeeinrichtung an der Instrumentenkomponente zu einem arbeitsfähigen Instrument zu ergänzen. Die Vorrichtung kann beispielsweise dazu eingerichtet sein, eine Elektrode zu tragen.

Insbesondere wenn die Vorrichtung eine Elektrode und eine Lichtaufnahmeeinrichtung aufweist, aber auch bei anderen Ausführungsformen, ist es bevorzugt, wenn die Vorrichtung derart eingerichtet ist, die räumliche Relativlage (Relativposition und/oder Relativausrichtung) der Elektrode und der Lichtaufnahmeeinrichtung relativ zueinander vorzugeben. Dabei kann nur eine einzige Relativposition und/oder Relativausrichtung ermöglicht werden oder es kann eine Relativposition und/oder Relativausrichtung aus mehreren möglichen Relativpositionen und/oder Relativausrichtungen auswählbar sein. Dabei kann zum Beispiel mindestens ein oder genau ein einziger Freiheitsgrad der Relativposition und/oder Relativausrichtung veränderbar sein. Damit können eine einzige oder mehrere Relativpositionen und/oder Relativausrichtungen zwischen der Lichtaufnahmeeinrichtung und der Elektrode vorgegeben werden. Beispielsweise kann die Elektrode in mehreren unterschiedlichen Drehlagen um ihre Längsachse an der Vorrichtung angeordnet werden.

Wenn die Elektrode beispielsweise eine Spatelelektrode ist, bei welcher zwischen zwei seitlichen Kanten auf gegenüberliegenden Seiten Spatelflächen angeordnet sind, können sich die eine oder die mehreren festgelegten Ausrichtungen von ausgeschlossenen Ausrichtungen dadurch unterscheiden, dass die Lichtaufnahmeeinrichtung beispielsweise in jeder der Ausrichtungen auf jeweils eine Spatelfläche oder in der einzigen Ausrichtung auf eine Spatelfläche schräg ausgerichtet ist. Die Vorrichtung kann mit einem Handgriff verbindbar sein und dazu eingerichtet sein, so den Handgriff zu einem mittels der Elektrode, welche in die Vorrichtung integriert ist oder eine eigenständige Komponente darstellt, arbeitsfähigen Instrument zu ergänzen. Der Handgriff ist bevorzugt mit einer Elektrode auch zu einem arbeitsfähigen Instrument ergänzbar, ohne die Lichtaufnahmeeinrichtung mittels der Vorrichtung an dem Instrument befestigen zu müssen. Die Instrumentenkomponente, beispielsweise der Handgriff, selbst ist folglich bevorzugt dazu eingerichtet, eine Elektrode zu tragen.

Die Vorrichtung legt bevorzugt eine vorbestimmte diskrete, **z.B.** diskrete Werte und/oder voneinander getrennte Bereiche, und/oder, zumindest bereichsweise, kontinuierliche Auswahl an möglichen räumlichen Relativlagen der befestigten Lichtaufnahmeeinrichtung relativ zu dem Instrument fest. Alle Relativlagen der Auswahl zeichnen sich dadurch aus, dass sichergestellt ist (wenn Vorrichtung, Instrument und Lichtaufnahmeeinrichtung für einander bestimmt sind), dass eine ausreichende Lichtmenge der Lichterscheinung (z.B. ein Funke oder ein fortwährend aufrechterhaltenes Plasma) hervorgerufen durch das Instrument von der Lichtaufnahmeeinrichtung aufnehmbar ist. Dadurch kann sichergestellt werden, dass der Arzt oder sein Assistent die Lichtaufnahmeeinrichtung stets lagerichtig am Instrument befestigt, so dass eine ausreichende Lichtmenge von der Lichtaufnahmeeinrichtung aufgenommen wird, um eine aussagekräftige Lichtanalyse zu gewährleisten. Die Auswahl kann eine einzige mögliche Relativlage oder mehrere mögliche Relativlagen einschließen. Die räumliche Relativlage wird durch die Position und Orientierung der Lichtaufnahmeeinrichtung, insbesondere des Lichteingangs der Lichtaufnahmeeinrichtung, relativ zu dem Instrument oder der Instrumentenkomponente des Instruments, beispielsweise der Elektrode oder dem Handgriff, bestimmt.

Die Auswahl kann eine diskrete Auswahl möglicher Relativlagen und/oder eine nicht-diskrete (kontinuierliche) Auswahl möglicher Relativlagen einschließen. Ausführungsformen sind möglich, in denen die Vorrichtung nur eine diskrete Auswahl einer endlichen Anzahl an vorgegebenen Relativlagen erlaubt. Alternativ sind Ausführungsformen möglich, in denen die Vorrichtung keine diskrete Unterauswahl festlegt, sondern eine nicht-diskrete Auswahl oder Einstellung von Relativlagen möglich ist. Eine nicht-diskrete Auswahl möglicher Relativlagen bedeutet insbesondere, dass der Anwender aus einer aktuellen Relativlage heraus die Relativlage verändern kann - und zwar über Spiel innerhalb der aktuellen Relativlage hinaus. Im Zuge dessen können kontinuierlich weitere mögliche Relativlagen durchlaufen werden.

Die räumliche Relativlage der befestigen Lichtaufnahmeeinrichtung wird relativ zu dem Instrument mittels der Vorrichtung vorzugsweise vollständig festgelegt (Relativlage einer diskreten Auswahl oder Unterauswahl an einer oder mehreren Relativlagen) oder beispielsweise bis auf einen oder zwei Freiheitsgrade (Relativlage in einer kontinuierlichen Unterauswahl möglicher Relativlagen). Die Vorrichtung kann die räumliche Relativlage beispielsweise bis auf einen oder zwei Winkel festlegen. Der Freiheitsgrad oder einer der Freiheitsgrade, die nicht oder nur bis auf eine Auswahl eines Wertebereichs festgelegt sind, kann beispielsweise der Winkel der Lichtaufnahme um das Instrument, beispielweise um eine Elektrode des Instruments, herum sein. Die Vorrichtung kann die räumliche Relativlage beispielsweise vollständig festlegen (nur eine einzige Relativlage ist möglich), wobei dies ein geringfügiges Spiel des Instruments in der oder bezüglich der Vorrichtung, beispielsweise in einer Längsrichtung des Instruments oder der Vorrichtung, einer Radialrichtung und/oder einer Drehrichtung, einbeziehen kann, welches die Lichtanalyse nicht beeinträchtigt. Jedenfalls aber wird eine Beweglichkeit der Lichtaufnahmeeinrichtung relativ zu dem Instrument, beispielsweise relativ zu einer Elektrode des Instruments, mittels der Vorrichtung derart eingeschränkt, dass in allen verbleibenden Lagen der Lichtaufnahmeeinrichtung relativ zu dem Instrument, beispielsweise relativ zu der Elektrode, eine zuverlässige Lichtaufnahme aus dem Bereich, in welchem die Funken und/oder das Plasma auf Grund der Einwirkung des Instruments entsteht, durch die Lichtaufnahmeeinrichtung gewährleistet ist. Bevorzugt umfasst oder umschließt der Lichtakzeptanzkegel in allen Lagen, welche die Vorrichtung der Lichtaufnahmeeinrichtung und dem Instrument relativ zueinander ermöglicht, den Bereich, in welchem die Lichterscheinungen erzeugt werden.

Mit Ausführungsformen der erfindungsgemäßen Vorrichtung kann eine Lichtaufnahmeeinrichtung an einem Instrument für die offene Chirurgie, zum Beispiel einem HF-Handgriff mit einer HF-Elektrode (auch (HF-)Applikator genannt), einem Instrument für die Laparoskopie oder einem Instrument für die flexible Endoskopie, wie zum Beispiel flexible Sonden für die Argon-Plasma-Koagulation, verwendet werden.

Wenn die Vorrichtung eine vorbestimmte Auswahl an räumlichen Relativlagen der befestigten Lichtaufnahmeeinrichtung relativ zu dem Instrument festlegt, ist eine Fehlausrichtung der Lichtaufnahmeeinrichtung durch den Anwender des Instruments ausgeschlossen, der als Arzt im medizinischen Gebrauch des Instruments geschult ist, nicht jedoch im Auffinden einer Lage der Lichtaufnahmeeinrichtung relativ zu dem Instrument, so dass die Lichtaufnahmeeinrichtung arbeiten kann. Ein Eingriff des Arztes mittels eines HFchirurgischen Instruments kann beispielsweise zunächst das Eröffnen und Freilegen des OP-Situs mittels eines HFchirurgischen Schneidinstruments bedeuten und danach die Präparation für die Gewebedifferenzierung. Beim Eröffnen und Freilegen des OP-Situs können teils Einstellungen der elektrischen Parameter für Schneiden erforderlich sein, welche eine effiziente Blutstillung möglich machen, aber auch eine große thermische Schädigung. Bei der Gewebedifferenzierung wird dagegen nur eine geringere thermische Schädigung akzeptiert. Auch kann sich die Elektrodenform unterscheiden, welche der Chirurg zum Eröffnen und Freilegen und zum Gewebedifferenzieren einsetzt. Die thermische Schädigung des Gewebes (höherer Energieeintrag) korreliert mit der Verschmutzung der optischen Faser am distalen Ende. Mit Ausführungsformen der erfindungsgemäßen Vorrichtung kann der Chirurg die Lichtaufnahmeeinrichtung mittels der Vorrichtung erst dann am Handgriff befestigen, wenn die Schritte der Eröffnung und Freilegen des OP-Situs abgeschlossen sind. Wenn für die anschließende Gewebedifferenzierung eine Einstellung des Instruments mit geringerer thermischer Schädigung erforderlich ist, ist die Gefahr des Verschmutzens des Lichteingangs vermindert.

Bevorzugt weist das Instrument eine mit HF-Energie beaufschlagbare Elektrode auf, so dass das Instrument unabhängig von der Vorrichtung für einen HFchirurgischen Eingriff verwendet werden kann. Die Elektrode des Instruments, beispielsweise des HF-Applikators, kann austauschbar sein. Das Instrument kann beispielsweise ein HF-Applikator mit einem Handgriff sein, welcher eine Elektrode aufweist. Die Elektrode kann an dem Handgriff austauschbar befestigbar sein. Der Handgriff kann die Position und Orientierung der Elektrode bis auf eine einzige oder eine diskrete Auswahl von Möglichkeiten in Bezug auf den Handgriff zu lassen. Alternativ oder zusätzlich kann die Vorrichtung beispielsweise sowohl die Lichtaufnahmeeinrichtung als auch eine Elektrode aufweisen.

Die Vorrichtung ist bevorzugt dazu eingerichtet eine räumliche Relativlage der befestigten Lichtaufnahmeeinrichtung relativ zu dem Instrument festzulegen, indem die Vorrichtung eine vorbestimmte Auswahl an räumlichen Relativlagen der befestigten Lichtaufnahmeeinrichtung relativ zu der Elektrode festlegt. Die Vorrichtung kann dazu eingerichtet sein, eine kontinuierliche oder diskrete Auswahl möglicher Lagen der Lichtaufnahmeeinrichtung relativ zu der Elektrode festzulegen. Die Vorrichtung kann dazu eingerichtet sein, eine einzige mögliche räumliche Relativlage für die befestigte Lichtaufnahmeeinrichtung relativ zu der Elektrode festzulegen. Durch die Festlegung einer Auswahl an möglichen Relativlagen soll Orientierung und Abstand der Lichtaufnahmeeinrichtung relativ zu einem Bereich an der Elektrode festgelegt werden, in welchem das Instrument mittels der Elektrode Lichterscheinungen erzeugt. Wenn die Orientierung und der Abstand der Lichtaufnahmeeinrichtung, insbesondere des Lichteingangs der Lichtaufnahmeeinrichtung, relativ zu der Elektrode vorbestimmt festgelegt ist, kann eine bestimmte Aussagekraft und Qualität der Lichtanalyse mittels der Lichtaufnahmeeinrichtung sichergestellt werden.

Die Vorrichtung weist bevorzugt eine Aufnahme auf, welche die Beweglichkeit des Instruments in der Aufnahme auf eine Beweglichkeit in eine Richtung, insbesondere eine Axialrichtung des Instruments, der Elektrode, der Aufnahme und/oder der Vorrichtung, einschränkt. Bevorzugt legt die Vorrichtung mittels der Aufnahme fest, dass das Instrument beim Befestigen in der Richtung, insbesondere Axialrichtung, in die Aufnahme einzuführen ist. Die Aufnahme kann dazu eingerichtet sein, die Bewegung des Instruments oder der Instrumentenkomponente in Axialrichtung in Richtung zum distalen Ende der Vorrichtung hin zu begrenzen.

Die Vorrichtung weist bevorzugt eine Einrichtung zum anschließenden Fixieren der axialen Position des Instruments in der Aufnahme auf, bis höchstens auf ein axiales Spiel, welches die Lichtanalyse nicht beeinträchtig, z.B. weil die Spitze der Elektrode trotz des Spiels stets in einem Lichtakzeptanzkegel der Lichtaufnahmeeinrichtung bleibt. Die Einrichtung zum anschließenden Fixieren kann dazu eingerichtet sein, die Bewegung des Instruments oder der Instrumentenkomponente in Axialrichtung in Richtung vom distalen Ende der Vorrichtung weg zu begrenzen.

Die Einrichtung zum anschließenden Fixieren der axialen Position weist bevorzugt einen Abschnitt auf, welcher seitlich relativ zur Axialrichtung des Instruments und/oder der Vorrichtung und/oder der Elektrode im Zuge der Befestigung der Lichtaufnahmeeinrichtung an dem Instrument beweglich ist. Dieser bewegliche Abschnitt ist bevorzugt dazu eingerichtet, nach dem Anordnen des Instruments in der Aufnahme durch seitliche Bewegung des Abschnitts in Eingriff mit dem Instrument gebracht zu werden, um die axiale Position des Instruments in der Aufnahme zu fixieren. Der bewegliche Abschnitt ist vorzugsweise flexibel mit der übrigen Vorrichtung befestigt, um den Abschnitt seitlich bewegen zu können. Dies wird gegenüber Ausführungsformen bevorzugt, bei denen der bewegliche Abschnitt an einem weiteren Abschnitt des Instruments angelenkt bzw. anscharniert ist. Bevorzugt ist der Abschnitt elastisch, so dass dieser nach dem Bewegen des Abschnitts gegen eine Federkraft vor dem Anordnen des Instruments in der Vorrichtung auf Grund der Federkraft sich selbstständig wieder in Richtung zu seiner Ausgangslage zurückbewegt. Mit flexiblem Abschnitt kann auf ein Scharnier verzichtet werden, was die Herstellung der Vorrichtung vereinfacht und die Vorrichtung einfacher zu sterilisieren und/oder zu säubern macht.

Die Vorrichtung weist bevorzugt einen Spülkanal auf, wobei der Spülkanal dazu eingerichtet ist, ein Spülmedium, beispielsweise Kohlenstoffdioxid, Edelgas, Stickstoff oder ein anderes chemisch inertes Gas oder Gasgemisch, bevorzugt laminar, entlang der Lichtaufnahmeeinrichtung an dem Lichteingang der Lichtaufnahmeeinrichtung vorbei auszugeben. Damit kann Verunreinigung des Lichteingangs der Lichtaufnahme verhindert werden, der Lichteingang der Lichtaufnahme gereinigt, Aerosol zwischen Lichteingang und Entstehungsort des Lichtes weggeblasen und/oder das Instrument, insbesondere eine Elektrode, gekühlt werden.

Bevorzugt ist die Vorrichtung dazu eingerichtet, die räumliche Lage des Spülkanals relativ zu der Lichtaufnahme auf eine, insbesondere diskrete, Auswahl räumlicher Relativlagen oder eine einzige räumliche Relativlage festzulegen. Der Spülfluss ist vorzugsweise in die Richtung ausgerichtet, in welche auch die Lichtaufnahmeeinrichtung bzw. deren Lichteingang schaut, d.h. in die Richtung der Mittelachse des Lichtakzeptanzkegels. In Ausführungsformen kann der Spülfluss an dem Lichteingang oder zwischen dem Lichteingang und dem Einwirkungsbereich der HF-Elektrode schräg auch bis 90° Grad zur optischen Achse, zum Beispiel durch eine Prallplatte oder durch eine einseitige Erhebung im Strömungskanal abgelenkt werden, um als Partikelbarriere zu dienen.

Bei zu starkem Strom an Spülmedium kann damit Gewebe verdrängt werden. Für eine hohe Präzision ist dies insbesondere bei der Präparation, also der präzisen Freistellung oder Freilegung von anatomischen Strukturen zu vermeiden. Andererseits soll der Volumenstrom an Spülmedium ausreichen, um Verschmutzung des Lichteingangs zuverlässig zu vermeiden und/oder um für eine ausreichende Kühlung der Elektrode zu sorgen. Des Weiteren können mit ausreichender Strömung gasförmige oder flüssige Medien beispielhaft Rauchgas, Blut oder flüssiges Fett verdrängt werden. Bei festgelegter räumlicher Lage des Spülkanals relativ zu der Lichtaufnahmeeinrichtung, insbesondere deren Lichteingang, kann mit genau abgestimmtem Volumenstrom an Spülmedium gearbeitet werden, welcher nicht zu einer präzisionsvermindernden Gewebeverdrängung führt, aber zu einer zuverlässigen Fernhaltung zu Verunreinigungen von dem Lichteingang und/oder Verdrängung von lichtaufnahmestörenden Medien und/oder Kühlung.

Vorzugsweise ist der Lichteingang der Lichtaufnahme in der Vorrichtung angeordnet, um Schutz des Lichteingangs vor Verschmutzungen zu bieten. Die Vorrichtung kann dadurch den Lichteingang gegen aus bestimmten Winkeln eintreffende Flüssigkeitstropfen oder Partikel abschirmen, ohne den Lichteinfall in den Lichteingang aus anderen Winkeln zu beeinträchtigen. In Ausführungsformen mit einem Spülkanal innerhalb der Vorrichtung kann der Lichteingang beispielsweise innerhalb des Spülkanals angeordnet sein bzw. die Lichtaufnahmeeinrichtung in dem Spülkanal enden.

Die Lichtaufnahme kann in Ausführungsformen mit Spülkanal beispielsweise innerhalb des Spülkanals oder durch den Spülkanal verlaufen. Gleichzeitig ist die Lichtaufnahmeeinrichtung bevorzugt innerhalb der Vorrichtung in einer einzigen bestimmten räumlichen Lage fixiert.

Eine besonders kompakte Bauform kann erhalten werden, wenn die Lichtaufnahmeeinrichtung - zum Beispiel ein gerader Endabschnitt der Lichtaufnahmeeinrichtung, welcher an den Lichteingang angrenzen kann - und/oder die optische Achse der Lichtaufnahmeeinrichtung und/oder die Mittelachse des Lichtakzeptanzkegels der Lichtaufnahmeeinrichtung und die Elektrode einen spitzen Winkel, beispielsweise kleiner 15°, einschließen.

In Ausführungsformen der Vorrichtung ist - bei hergestellter Verbindung zwischen der Vorrichtung und dem Instrument - bevorzugt ein Kanalabschnitt des Spülkanals ausgebildet, welcher einerseits von einer Wand der Vorrichtung und andererseits von einer ersten Längsseite einer Elektrode und/oder eines Elektrodenschafts der Vorrichtung begrenzt ist. Der Kanalabschnitt ist von Spülmedium durchströmbar. Eine der ersten Längsseite gegenüberliegende zweite Längsseite der Elektrode und/oder des Elektrodenschafts liegt entlang des Kanals vorzugsweise wenigstens abschnittsweise frei. Während das Spülmedium durch den Kanalabschnitt strömt, kann dieses die Elektrode an der ersten Längsseite kühlen. Die zweite Längsseite liegt frei, hat also insbesondere Kontakt zu der Umgebung der Vorrichtung, insbesondere Luftkontakt oder Kontakt zum gas- oder aerosolförmigen Umgebungsmedium, und kann dadurch genügend Wärme abgeben, so dass sich die Elektrode bei Betrieb insgesamt nicht übermäßig erwärmt. Der Volumenstrom an Spülmedium kann damit auf eine Rate eingestellt werden, die eine ausreichende Kühlung der Elektrode - und bevorzugt auch eine ausreichende Abschirmung und/oder Reinigung des Lichteingangs vor Verschmutzung - gewährleistet.

Die Lichtaufnahmeeinrichtung, beispielsweise eine optische Faser, erstreckt sich vorzugsweise von dem Lichteingang aus in der und/oder neben der Vorrichtung durchgehend - ohne optische Schnittstelle zwischendurch - proximal über die Vorrichtung hinaus, um Licht zur Analyse weiterzuleiten. Dies ist von Vorteil, da eine optische Schnittstelle verschmutzen kann, wenn an ihr für einen Teil des operativen Eingriffs keine Lichtaufnahme mit Lichteingang angeschlossen wäre.

Bei dem Instrument kann es sich beispielsweise um ein HF-chirurgisches Schneid-, Koagulations- und/oder Devitalisierungsinstrument handeln. Das Instrument kann beispielsweise dazu eingerichtet sein, mit einem ionisierbaren Gas zu arbeiten, welches in einen Plasmazustand überführt wird. Das Instrument kann insbesondere ein Argon-Plasma-Koagulations (APC)- Instrument sein. Das Instrument kann alternativ oder zusätzlich dazu eingerichtet sein, eine dielektrische Barriereentladung zu nutzen.

Es wird erfindungsgemäß außerdem eine Anordnung aus einem Instrument und einer erfindungsgemäßen Vorrichtung derart angegeben, dass in der Anordnung mittels der Vorrichtung eine Lichtaufnahmeeinrichtung an dem Instrument befestigt ist.

Weitere Merkmale und vorteilhafte Ausführungsformen der Vorrichtung und der Anordnung ergeben sich aus der nachfolgenden Beschreibung sowie den Figuren. Es zeigen beispielhaft:
Figur 1a - eine perspektivische Darstellung eines Instruments in Form eines HF-Applikators, welcher in einem Ausführungsbeispiel einer erfindungsgemäßen Vorrichtung befestigt angeordnet ist,
Figur 1b - die erfindungsgemäße Vorrichtung gemäß Figur 1a in einer längsgeschnittenen perspektivischen Ansicht,
Figur 2a - die beispielhafte erfindungsgemäße Vorrichtung gemäß Figuren 1a und 1b in einer seitlichen Ansicht mit ausschnittsweiser Längsschnittdarstellung des distalen Endabschnitts der Vorrichtung,
Figur 2b - eine Vergrößerung der ausschnittsweisen Längsschnittdarstellung aus Figur 2a (Ausschnitt in Figur 2a mit einem Kreis veranschaulicht),
Figur 3a - das Ausführungsbeispiel der erfindungsgemäßen Vorrichtung gemäß Figuren 1a und 1b in einer Draufsicht,
Figur 3b - eine ausschnittsweise Draufsicht auf das distale Ende der Vorrichtung gemäß Figuren 1a und 1b (Ausschnitt in Figur 3a mit einem Kreis veranschaulicht),
Figur 4a - eine Seitenansicht des Ausführungsbeispiels des Instruments gemäß Figur 1a in Form eines HF-Applikators, welcher in dem Ausführungsbeispiel der erfindungsgemäßen Vorrichtung gemäß Figur 1a befestigt angeordnet ist,
Figur 4b - eine Draufsicht des Ausführungsbeispiels des Instruments gemäß Figur 1a befestigt in der Vorrichtung gemäß Figur 1a,
Figur 4c - eine längsgeschnittene Ansicht des Instruments in der Vorrichtung gemäß Figur 1a,
Figur 4d - einen Ausschnitt der Längsdarstellung gemäß Figur 4c,
Figur 4e - eine Ausrichtstruktur und eine Gegenausrichtstruktur,
Figuren 5a und 5b - stark schematisierte Darstellungen von möglichen relativen Ausrichtungen eines Lichteingangs zu einer Elektrode und
Figur 6 - ein weiteres Ausführungsbeispiel einer erfindungsgemäßen Vorrichtung mit einem verbundenen Beispiel eines Instruments für die laparoskopische Chirurgie,
Figur 7 - ein weiteres Ausführungsbeispiel einer erfindungsgemäßen Vorrichtung mit einem verbundenen Beispiel eines Instruments für die endoskopische Chirurgie,
Figur 8a bis 10b - ein weiteres Ausführungsbeispiel einer erfindungsgemäßen Vorrichtung mit einer Absaugeinheit,
Figur 11a - ein Handgriffteil eines Instruments,
Figur 11b - eine erfindungsgemäße Vorrichtung, welche eine Elektrode trägt,
Figur 11c - einen Ausschnitt der Längsdarstellung gemäß Figur 11b,
Figur 12a - eine erfindungsgemäße Vorrichtung mit einem Verlängerungselement zwischen einer Elektrode und einem Handgriff in einer perspektivischen Ansicht,
Figur 12b - die Anordnung gemäß Figur 12a in einer Längsschnittansicht,
Figur 13 eine perspektivische Teildarstellung in einem Bereich im Anschluss an ein distales Ende eines Instruments, das in einem weiteren Ausführungsbeispiel einer erfindungsgemäßen Vorrichtung angeordnet ist und
Figur 14 einen Längsschnitt durch den in Figur 13 dargestellten Bereich des Instruments und der Vorrichtung.

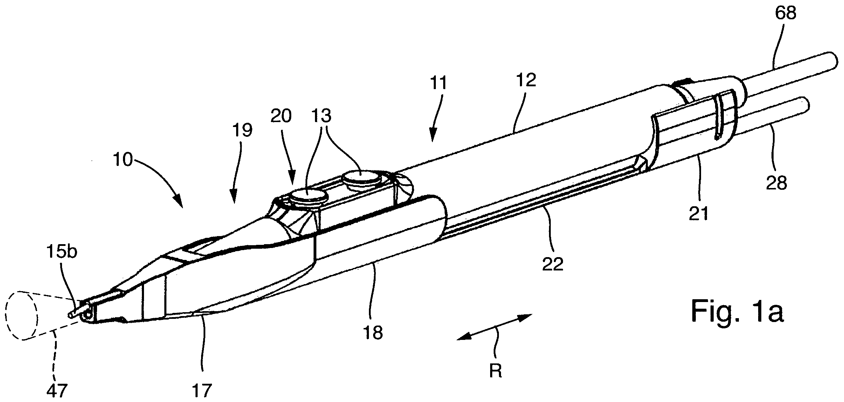

Figur 1a zeigt in einer perspektivischen Ansicht eines Ausführungsbeispiel einer erfindungsgemäßen Vorrichtung 10 in Form eines Handgriffteils, in welchem ein beispielhaftes Instrument 11, hier ein Applikator für die offene Chirurgie, aufgenommen ist. Das Instrument 11 ist bevorzugt ein Instrument zu monopolaren HF-Chirurgie, bei welcher ein Pol der Spannungsquelle an einer großflächigen, am Patienten befestigten sogenannten Neutralelektrode angeschlossen ist (nicht dargestellt). Alternativ kann das Instrument 11 ein Instrument für die bipolare HF-Chirurgie sein.

Der das Instrument 11 darstellende Applikator selbst weist ein Handgriffteil 12 mit Bedienelementen 13 auf. In dem distalen Endabschnitt des Handgriffteils 12 ist ein Elektrodenaufnahmeschaft 14 mit einer Elektrode 15 aufgenommen. Das Handgriffteil 12 ist mit einer elektrischen Leitung 68 verbunden, über welche die Elektrode 15 mit elektrischer Hochfrequenzleistung beaufschlagt werden kann.

Die Vorrichtung 10 weist einen distalen Abschnitt 17 auf, in welchem der Applikator 11 steckt. Der proximale Endabschnitt 18 des distalen Abschnitts 17 der Vorrichtung 10 weist eine Halbschalenform auf, welche den Applikator 11 aufnimmt. Die Halbschalenform 18 ist an einer Längsseite 19 (Oberseite) offen, um einen Zugang zu den Bedienelementen 13 des Instruments 11 an der Oberseite 20 des Instruments 11 bereitzustellen.

Die Vorrichtung 10 weist außerdem einen beispielsgemäß halbschalenförmigen proximalen Abschnitt 21 auf. Dieser ist an der Längsseite 19 der Vorrichtung 10 offen, an welcher auch der proximale Endabschnitt 18 offen ist. Der proximale Abschnitt 21 ist über einen flexiblen Übergangsabschnitt 22 mit dem distalen Abschnitt 17 der Vorrichtung 10 verbunden. Die Vorrichtung 10 umschließt den Applikator 11 zumindest abschnittsweise formschlüssig, um die Relativlage einer Lichtleiteinrichtung und/oder Lichtaufnahmeeinrichtung 24 relativ zu dem Applikator 11 festzulegen. Die Lichtleiteinrichtung und/oder Lichtaufnahmeeinrichtung kann insbesondere wenigstens eine optische Faser aufweisen.

Die Vorrichtung 10 erstreckt sich vom proximalen Ende zum distalen Ende im Wesentlichen in einer Axialrichtung R. Der distale Abschnitt 17 und/oder der proximale Abschnitt 21 können einen sich um eine parallel zur Längsrichtung L erstreckende Krümmungsachse gekrümmten Verlauf aufweisen. Beispielsgemäß hat der Übergangsabschnitt 22 eine Stabform. Der Übergangsabschnitt 22 ist mit dem distalen Abschnitt 17 und/oder dem proximalen Abschnitt 21 an einer zentralen Stelle verbunden, ausgehend von der sich zwei Schalenteile des distalen Abschnitts 17 bzw. des proximalen Abschnitts 21 in entgegengesetzte Richtungen krümmen, um die jeweilige Halbschale zu bilden. Insbesondere bildet der Übergangsabschnitt 22 - im Unterschied zum distalen Abschnitt 17 und zum proximalen Abschnitt 21 - keinen konkaven Aufnahmebereich für den Applikator 11.

Der distale Abschnitt 17, der Übergangsabschnitt 22 und der proximale Abschnitt 21 bestehen vorzugsweise aus dem gleichen Kunststoff. Der Übergangsabschnitt 22 ist mit dem distalen Abschnitt 17 und dem proximalen Abschnitt 21 vorzugsweise nahtlos einstückig verbunden. Die Vorrichtung 10 kann insbesondere im Spritzgießverfahren hergestellt sein. Die Flexibilität des Übergangsabschnitts 22 verglichen mit dem distalen Abschnitt 17 und/oder dem proximalen Abschnitt 21 ist beispielsgemäß dadurch erreicht, dass der Übergangsabschnitt 22 schlanker als der distale Abschnitt 17 und der proximale Abschnitt 21 ausgebildet ist. Er kann insbesondere in jede Richtung quer zu seiner Erstreckung eine die Flexibilität gewährleistende geringe Dimension aufweisen. Bevorzugt ist die Vorrichtung 10 oder zumindest der distale Abschnitt 17 transparent, um eine verbesserte Sicht auf den OP-Situs zu schaffen.

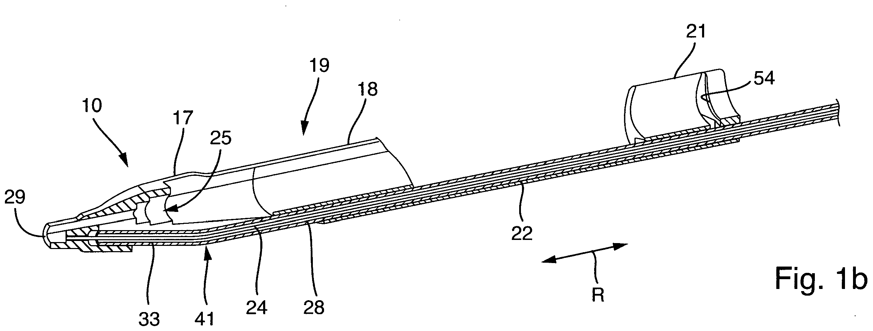

Wie insbesondere in Figuren 1b, 2b und 4d veranschaulicht, stellt der distale Abschnitt 17 der Vorrichtung 10 eine kanalartige erste Aufnahme 25 für einen Formschlussabschnitt 26 des Applikators 11 bereit. Die erste Aufnahme 25 (kann auch erster Aufnahmekanal genannt werden) ist vorzugsweise ringsum (in alle Radialrichtungen) geschlossen. Die erste Aufnahme 25 legt mittels Formschluss eine Ausrichtung der Elektrode 15 des Instruments 11 in der Vorrichtung 10 entlang einer Längsachse L der ersten Aufnahme 25 fest.

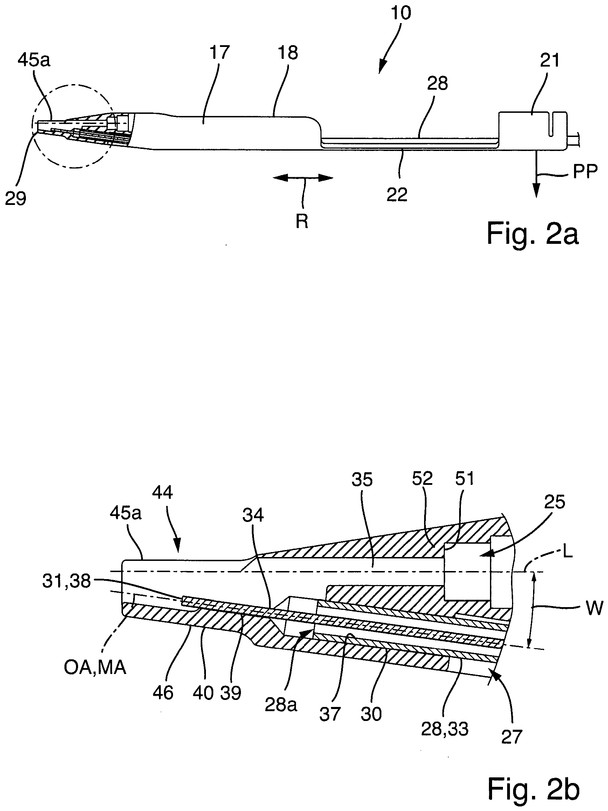

Die Vorrichtung 10 weist, wie beispielhaft insbesondere auch in den Figur 2b gezeigt, zudem eine abschnittsweise kanalartige zweite Aufnahme 27 für eine Leitung 28, beispielsweise einen Schlauch oder eine Röhre, auf, durch welche sich die wenigstens eine optische Faser, d.h. eine einzelne optische Faser oder ein Bündel an optischen Fasersträngen, als Lichtaufnahmeeinrichtung 24 in Richtung zu dem distale Ende 29 des distalen Abschnitts 17 der Vorrichtung 10 erstreckt. Die Leitung 28 ist in der zweiten Aufnahme 27 vorzugsweise durch Kraft- und/oder Reibschluss mit der Wandfläche 30 der zweiten Aufnahme 27 gegen Bewegen der Leitung 28 in der zweiten Aufnahme 27 fixiert. Die Leitung 28 bildet einen Leitungskanal (Spülkanal) und dient dem Leiten eines Spülmediums zum Reinigen und/oder Abschirmen eines Lichteingangs 31 der optischen Faser 24. Die optische Faser 24 dient der Aufnahme von Licht aus Lichterscheinungen, welche durch die Elektrode 15 des Applikators 11 erzeugt werden. Die Lage der optischen Faser 24 in der Vorrichtung 10 ist bevorzugt vollständig festgelegt.

Die Leitung 28 verläuft geschützt seitlich neben dem Applikator 11 zwischen dem flexiblen Übergangsabschnitt 22 und dem Applikator 11 durch den halbschalenförmigen proximalen Abschnitt 21 des distalen Abschnitts 17 der Vorrichtung 10.

Die zweite Aufnahme 27 für die Leitung 28 ist dazu eingerichtet, diese abschnittsweise im Wesentlichen parallel zu dem Applikator 11 in Richtung zu dem distalen Ende 29 der Vorrichtung 10 zu führen. Wie insbesondere in den Figuren 1b und 2b veranschaulicht, ist die zweite Aufnahme 27 ferner dazu eingerichtet, einen an das distale Ende 32 der Leitung 28 angrenzenden Leitungsabschnitt 33 der Leitung 28 in einem spitzen Winkel an die Längsachse L der ersten Aufnahme 25 heranzuführen. Ebenso schließt, wie insbesondere Figur 2b veranschaulicht, der an den Lichteingang 31 der optischen Faser 24 angrenzende Endabschnitt 34 der optischen Faser 24 bzw. dessen optische Achse OA und/oder die Mittelachse MA des Lichtakzeptanzkegels 47 der optischen Faser 24 einen spitzen Winkel W mit der Längsachse L der Aufnahmebohrung 35 der ersten Aufnahme 25 für die Elektrode 15 oder den Elektrodenaufnahmeschaft 14 ein - und damit auch mit der Längsachse ELA der Elektrode 15, welche mit der Längsachse L der ersten Aufnahme 25 zusammenfällt. Der spitze Winkel, in welchem der Leitungsabschnitt 33 an die Längsachse L der ersten Aufnahme 25 herangeführt wird, stimmt bevorzugt mit dem Winkel W zwischen Mittelachse MA des Lichtakzeptanzkegels 47 und der Längsachse ELA der Elektrode 15 überein.

Die optische Faser 24 kann in der Leitung 28 zentriert gehalten sein bzw. im Wesentlichen koaxial deren Verlauf folgen. Dazu können beispielsweise Halteelemente (nicht gezeigt) dienen, die innerhalb der Leitung 28 angeordnet sind, ein Spülmediumfluss durch die Leitung 28 an das distale Ende 29 der Vorrichtung 10 erlauben und gleichzeitig die optische Faser 24 auf Abstand zu der Innenwandfläche 37 der Leitung 28 halten. Alternativ kann die Faser 24 beispielsweise lose in dem Leitungsschlauch 28 liegen, wobei ein Endabschnitt 34 der Faser, wie weiter unten beschrieben, fest mit der Vorrichtung 10 verbunden sein kann. Die optische Faser 24 ist, beispielsweise mittels der Halteelemente, bevorzugt auch gegen Verdrehen der optischen Faser 24 um eine Längsachse der optischen Faser 24 gesichert. Der Volumenstrom an Spülmedium wird im Ausführungsbeispiel im Wesentlichen konzentrisch zur optischen Faser 24 geführt. Alternativ kann der Volumenstrom am distalen Ende 38 der optischen Faser 24 seitlich zugeführt werden. In beiden Ausführungsformen dient der das distale Ende 38 der optischen Faser 24 umspülende Spülstrom der Vermeidung von Verschmutzung durch verspritzte Gewebeflüssigkeitströpfchen oder umherfliegende Partikel auf dem Lichteingang 31 der optischen Faser 24.

Wie die Figuren 2a und 2b veranschaulichen, ragt die optische Faser 24 aus dem distalen Ende 32 der Leitung 28 heraus, so dass ein Endabschnitt 34 der optischen Faser 24 mit dem spitzem Winkel W2 zu dem Elektrodenaufnahmeschaft 14 neben diesem verläuft. Die optische Achse OA (z.B. die Längsachse der optischen Faser) und/oder die Mittelachse MA des Lichtakzeptanzkegels 47 schließen damit mit der Elektrodenlängsachse ELA den spitzen Winkel W2 von beispielsweise kleiner 15°, dargestellt 7,5°, ein. Der Endabschnitt 34 der optischen Faser 24, welcher aus dem distalen Ende 32 der Leitung 28 herausragt, kann dazu auf einem im Längsschnitt durch die Vorrichtung (Figur 2b) sockelartig oder vorsprungartig erscheinenden Abstützabschnitt 39 der Aufnahmewand 40 des distalen Abschnitts 17 der Vorrichtung 10 aufliegen und damit beispielsweise stoffschlüssig fest verbunden sein, um den Endabschnitt 34 der optischen Faser 24 in dieser Lage zu stabilisieren. Die optische Faser 24 wird durch deren Eigenspannung aufgrund der Biegung 41 der optischen Faser 24 in der Leitung 28 gegen den Abstützabschnitt 39 gedrückt. Damit ist die Lage der optischen Faser 24 relativ zu der Längsachse L der ersten Aufnahme 25 festgelegt. Dies gilt auch für die Richtung senkrecht zur Zeichenebene in Figur 2b. Der Aufnahmebereich 42 des Abstützabschnitts 39 kann beispielsweise halbschalenförmig sein, um die Lage der optischen Faser 24 zusammen mit der Eigenspannung der optischen Faser 24 quer zu der Ebene festzulegen, welche von dem Endabschnitt 34 der optischen Faser 24 und der Längsachse L (Axialrichtung) der ersten Aufnahme 25 bzw. der Axialrichtung der Elektrode 15 aufgespannt wird.

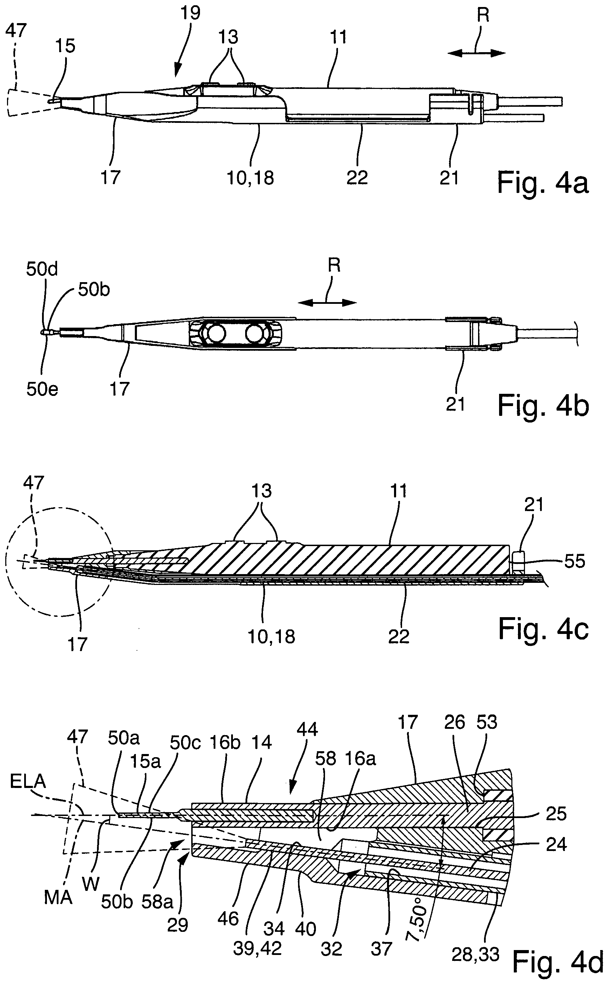

Um einer Verschmutzung der optischen Faser 24 entgegenzuwirken, endet die optische Faser 24 vorzugsweise nicht bündig mit der Vorrichtung 10 und steht auch nicht aus der Vorrichtung 10 hervor. Vielmehr endet die optische Faser 24, wie dargestellt Figuren 2b, 4d, vorzugsweise relativ zu dem distalen Ende 29 der Vorrichtung 10 zurückgesetzt innerhalb eines kanalförmigen Bereichs 44, um eine direkte Verschmutzung der optischen Faser 24 aus bestimmten Winkel zu vermeiden.

Der Bereich 44 der Vorrichtung 10 ist vorzugsweise zu einer Seite offen. Der Bereich 44 kann beispielsweise, wenn auch der proximale Endabschnitt 18 des distalen Abschnitts 17 offen ist (z.B. nach "oben" in beispielsweise den Figuren 1b und 2a), zu derselben Seite hin offen sein. Dies ergibt sich insbesondere aus den Figuren 1b, 2a und 2b, in denen die Vorrichtung 10 dargestellt ist, ohne dass eine Verbindung zwischen der Vorrichtung 10 und dem Instrument 11 hergestellt ist. Zu der gegenüberliegenden Seite ("nach unten") ist der Bereich 44 dagegen vorzugsweise geschlossen. Die vom proximalen Endabschnitt 18 des distalen Abschnitts 17 gebildete Halbschale kann in Ausführungsbeispielen durchbrechungsfrei sein und insbesondere seitlich geschlossen sein.

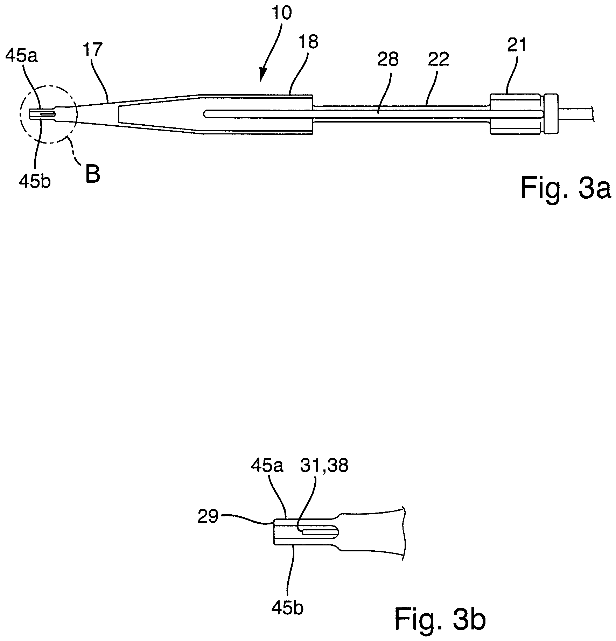

Die Vorrichtung 10 weist in dem Bereich 44 zwei Seitenwandabschnitte 45a, 45b auf, welche sich seitlich an der optischen Faser 24 vorbeierstrecken, so dass das distale Ende 38 der optischen Faser 24 relativ zu dem distalen Ende 29 der Vorrichtung 10 zurückversetzt ist. Damit wird der Lichteingang 31 an der Stirnseite der optischen Faser 24 zumindest vor Partikeln und/oder Tröpfchen geschützt, welche sich aus bestimmten Winkeln in Richtung Lichteingang 31 bewegen. Die Seitenwandabschnitte 45a, 45b sind über einen Bodenwandabschnitt 46 verbunden, so dass der Bereich 44 bzw. die Vorrichtung 10 dort einen beispielsweise U- oder V-förmigen Querschnitt aufweisen kann, wie es sich aus Figur 2b in Verbindung mit Figur 3a ergibt. Der Abstützabschnitt 39 wird zumindest teilweise von dem Bodenabschnitt 46 gebildet. Das freie Ende 38 der optischen Faser 24 kann über den Abstützabschnitt 39 etwas überstehen und damit in alle radialen Richtungen von den Wandabschnitten 45a, 45b, 46 der Vorrichtung 10 in dem Bereich 44 etwas beabstandet sein. Dies vermeidet eine zu große Abschattung des Lichtakzeptanzkegels 47 der optischen Faser 24 durch die Vorrichtung 10.

Der Lichtakzeptanzkegel 47 ist in den Figuren 1a, 4c sowie 4d zur Veranschaulichung mit gestrichelten Linien eingezeichnet. Der Lichtakzeptanzkegel 47 beinhaltet alle Richtungen, aus denen die optische Faser 24 in ihren Lichteingang 31 Licht aufnehmen und zu einer Analysevorrichtung weiterleiten kann, mit welcher das Licht analysiert werden kann. In dem Elektrodenaufnahmeschaft 14 ist ein Arbeitsabschnitt 15a der Elektrode 15 fixiert. Wie dargestellt enthält der Lichtakzeptanzkegel 47 die Spitze oder das distale Ende 50a der Elektrode 15 bzw. des Arbeitsabschnitts 15a. Die Position und/oder die Orientierung der Elektrode 15, insbesondere deren Arbeitsabschnitt 15a oder Spitze 50, relativ zu dem Formschlussabschnitt 26 des Instruments 11 ist eindeutig festgelegt. Zudem ist die Position und/oder die Orientierung der optischen Faser 24 relativ zu der ersten Aufnahme 25 eindeutig festgelegt. Der Formschlussabschnitt 26 sorgt zusammen mit der ersten Aufnahme 25 für eine definierte Lage der optischen Faser 24 relativ zu der Elektrode 15, insbesondere zu deren Arbeitsabschnitt 15a, bei Verbindung zwischen Vorrichtung 10 und Instrument 11. Mittels Formschluss zwischen der Vorrichtung 10 und dem Instrument 11 wird sichergestellt, dass die Position und/oder die Orientierung der optischen Faser 24 bei Verbindung zwischen der Vorrichtung 10 und dem Instrument 11 eine Lichtanalyse der Lichterscheinungen erlaubt, welche mittels des Arbeitsabschnitts 15a der Elektrode 15 erzeugt werden. In Ausführungsbeispielen kann die Position der Elektrode 15 relativ zu dem Instrument 11 und/oder der Vorrichtung festgelegt sein, jedoch die Orientierung der Elektrode 15 um deren Längsachse ELA innerhalb einer kontinuierlichen Auswahl frei wählbar sein.

Zu dem Formschlussabschnitt 26 des Applikators 11 kann beispielsweise ein Abschnitt der Elektrode 15 gehören. Zu dem Formschlussabschnitt 26 gehört im dargestellten Ausführungsbeispiel ein Abschnitt des Elektrodenaufnahmeschafts 14 der Elektrode 15 des Applikators 11. Der Formschlussabschnitt 26 weist eine Form auf, welche zu einer entsprechenden komplementären Gegenform der ersten Aufnahme 25 der Vorrichtung 10 passt. Im dargestellten Ausführungsbeispiel weist der Elektrodenaufnahmeschaft 14 eine zylindrische Form auf, welche in die zylindrische Form (Bohrung) der ersten Aufnahme 25 passt. Alternativ zu der zylindrischen Form des Formschlussabschnitts 26 und der ersten Aufnahme 25 können diese beispielsweise zueinander passende polyedrische Formen, **z.B.** eine Rechteckform, aufweisen. Mit dem Paar aus Formschlussabschnitt 26 und erster Aufnahme 25 wird die relative Beweglichkeit des Formschlussabschnitts 26 in der ersten Aufnahme 25 und bezüglich der ersten Aufnahme 25 in alle Richtungen senkrecht zu der Mittelachse MA der ersten Aufnahme 25 bzw. zur Längsachse LA des Elektrodenaufnahmeschaftes 14 und damit auch relativ zum Arbeitsabschnitt 15a der Elektrode 15 unterbunden. Mittels des ersten Aufnahmekanals 25, welcher in alle Radialrichtungen geschlossen sein kann, ist die Lage der Elektrode 15 relativ zu dem Endabschnitt 34 der optischen Faser 24 radial in alle Richtungen festgelegt.

Ein entsprechender erster Anschlag 51 (siehe insbesondere Figur 2b) wird durch einen Wandabschnitt 52 der Vorrichtung 10 gebildet. Dieser liegt einem ersten Gegenanschlag 53 (siehe insbesondere Figur 4d) des Applikators 11 axial gegenüber, wenn der Applikator 11 in die erste Aufnahme 25 vorgeschoben wird. Der erste Gegenanschlag 53 kann beispielsweise von den Elektrodenaufnahmeschaft 14 oder, wie dargestellt, von einer Elektrodenschaftumspritzung gebildet sein, welche ein Abschnitt des Handgriffteils 12 ist. Der erste Anschlag 51 und der ersten Gegenanschlag 52 dienen dazu, die Lage der optischen Faser 24 in Richtung der Elektrodenspitze 50a bzw. in axialer Richtung der Elektrode 15 bzw. des Elektrodenaufnahmeschaftes 14 festzulegen. Mittels des ersten Anschlags 51 und des ersten Gegenanschlags 52 wird der maximale Abstand zwischen Elektrodenspitze 50a und Lichteingang 31 festgelegt. Die Lage der optischen Faser 24 relativ zu der Elektrode 15 ist bevorzugt so festgelegt, dass die optische Faser 24 auf einen Bereich vor der Spitze 50a der Elektrode 15 schaut. Die optische Achse OA der optischen Faser 24 schneidet einen Bereich vor der Elektrodenspitze 50a - vorzugsweise die Längsachse ELA der Elektrode 15 in einem Punkt vor der Spitze 50a der Elektrode 15.

Ein zweiter Anschlag 54 zur axialen Sicherung und insbesondere Positionierung des Applikators 11 ist an dem proximalen Abschnitt 18 der Vorrichtung 10 ausgebildet. Ein zweiter Gegenanschlag 55 des Instruments 11 wird im Ausführungsbeispiel durch das proximale Ende des Applikators 11 gebildet (s. Figur 4c). Der zweite Anschlag 54 und der zweite Gegenanschlag 55 verhindern eine ungewollte Bewegung des Applikators 11 vom distalen Ende 29 der Vorrichtung 10 weg. Dadurch kann ein Mindestabstand zwischen der Elektrodenspitze 50a und dem Lichteingang 31 der optischen Faser 24 aufrechterhalten werden. Die Lage des Lichteingangs 31 der optischen Faser 24 relativ zu der Spitze 50a der Elektrode 15 ist mittels der Anschlag/Gegenanschlagpaare 51, 51, 54, 55 axial bis auf höchstens ein die Lichtanalyse mittels der optischen Faser 24 nicht beeinträchtigendes Spiel zwischen dem Instrument 11 und der Vorrichtung 10 in Axialrichtung R festgelegt. Insbesondere bleibt die Elektrodenspitze 50a bei hergestellter Verbindung zwischen der Vorrichtung 10 und dem Instrument 11 im Lichtakzeptanzkegel 47 der optischen Faser 24, wie Figuren 4c und 4d veranschaulichen. Der Lichtakzeptanzkegel 47 der optischen Faser 24 ist bevorzugt so ausgerichtet, dass die Spitze 50a der HF-Elektrode 15 derart im Zentrum des Lichtakzeptanzkegels 47 liegt, die Lichterscheinungen links und rechts (an der einen wie auch an der gegenüberliegenden Schmalseite) um die Elektrode 15 zu erfassen.

Um möglichst viel Licht von dem HF-Funken zu erfassen - dieser stellt eine divergente Lichtquelle dar -, sollte der Durchmesser des Lichteingangs 31 der optischen Faser 24 möglichst groß sein und der Lichteingang 31 möglichst nah positionstreu zum Entstehungsort der Lichterscheinung, beispielsweise einem HF-Funken, an der Elektrodenspitze 50a positioniert werden. Um eine Verschmutzung der optischen Faser 24 durch zum Beispiel Rauchpartikel, Aerosole oder durch wegspritzende Gewebepartikel, zum Beispiel Fetttröpfchen, entgegenzuwirken, müsste der Lichteingang 31 der optischen Faser 24 dagegen möglichst weit von dem Entstehungsort der Lichterscheinung sein. Mit der Festlegung der Position des Applikators 11 zwischen dem ersten Anschlag 51 und dem zweiten Anschlag 54 an der und relativ zur Vorrichtung 10 ist einerseits eine hohe Lichtaufnahme und andererseits eine höchstens geringe Verschmutzung sichergestellt.

Der Arbeitsabschnitt 15a der Elektrode 15 kann beispielsweise spatelförmig (wie dargestellt) oder nadelförmig sein. Die Elektrode 15 ragt aus dem distalen Ende 29 der Vorrichtung 10 heraus bzw. steht über das distale Ende 29 der Vorrichtung 10 in distale Richtung über. Die Elektrode 15 ist beispielsweise so ausgerichtet, dass eine flache Seite (diese kann auch als Flachseite oder Spatelseite bezeichnet werden) 50b der Elektrode 15 dem Lichteingang 31 der optischen Faser 24 schräg zugewandt ist. Alternativ kann die Elektrode 15 derart ausgerichtet sein, dass dem Lichteingang 31 eine schmale Seite (kann auch als Schmalseite oder Kante bezeichnet werden) 50d, 50e der Elektrode 15 zugewandt ist. Die Drehorientierung der Vorrichtung 10 bzw. der optischen Faser 24 um die Längsachse ELA der Elektrode 15, beispielsweise die Längsachse des spatelförmigen Arbeitsabschnitts 15a, kann beispielsweise bei Verbindung des Instruments 11 mit der Vorrichtung 10 beispielsweise mittels einer Ausrichtstruktur 56 und einer Gegenausrichtstruktur 57 festgelegt werden.

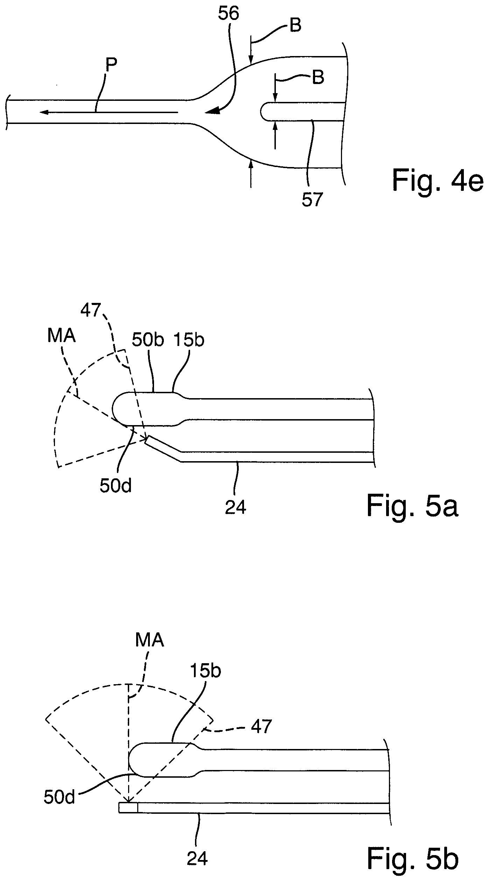

Eine beispielhafte Ausrichtstruktur 56 und Gegenausrichtstruktur 57 sind in Figur 4e gezeigt. Ein Paar aus einer Ausrichtstruktur 56 und einer Gegenausrichtstruktur 57 kann beispielsweise aus einer Ausnehmung 56 und einem Fortsatz 57 bestehen, wobei der Fortsatz 57 relativ zu der Ausnehmung 56 in die Ausnehmung 56 zu schieben ist. Die Ausnehmung 56 und/oder der Fortsatz 57 können, wie dargestellt, derart ausgebildet sein, dass deren Breite B an dem Eingang der Ausnehmung 56 in Schieberichtung abnimmt. Eine Ausrichtstruktur 56 an dem Umfang des Applikators 11 gerät beispielsweise beim Vorschieben der Elektrode 15 in die erste Aufnahme 25 mit einer Gegenausrichtstruktur 57 der Vorrichtung 10 in Eingriff, so dass die Ausrichtung der optischen Faser 24 um die Längsachse ELA der Elektrode 15 beim Verbinden des Instruments 11 mit der Vorrichtung 10 festgelegt wird. Selbstverständlich kann die Ausrichtstruktur 56 alternativ an der Vorrichtung 10 und die Gegenausrichtstruktur 57 an dem Instrument 11 ausgebildet sein. Der Pfeil P veranschaulicht die Schieberichtung, in die das Instrument 11 in die Vorrichtung 10 beim Herstellen der Verbindung vorgeschoben wird.

Beim Zusammensetzen der Vorrichtung 10 und des Handgriffs 11 während der Operation kann der Verwender der Vorrichtung, beispielsweise ein Chirurg oder ein Assistent des Chirurgen, beispielsweise wie folgt vorgehen: Der Verwender drückt den proximalen Abschnitt 21 der Vorrichtung 10 von einer Längsachse des distalen Abschnitts 17 bzw. der Längsachse L der ersten Aufnahme 25 gegen eine Federkraft des Übergangsabschnitts 22 seitlich weg (siehe Pfeil PP in Figur 2a), so dass der Verwender die Elektrode 15 nun auf der Längsachse L in die erste Aufnahme 25 schieben kann. Da die erste Aufnahme 25 den Elektrodenaufnahmeschaft 14 passgenau aufnimmt, ist es ohne seitliches Wegdrücken des proximalen Abschnitts 21 nicht möglich, den Applikator 11 so relativ der Vorrichtung 10 auszurichten, dass die Elektrode 15 in die erste Aufnahme 25 geschoben werden kann. Spätestens wenn der erste Gegenanschlag 52 und der erste Anschlag 51 aneinander anstoßen, kann der Verwender durch Freigeben der Gegenbewegung des proximalen Abschnitts 21, welche Gegenbewegung der proximale Abschnitt 21 auf Grund der Federkraft automatisch ausführt, den Applikator 11 in der Vorrichtung axial zwischen dem ersten Gegenanschlag 52 und dem zweiten Gegenanschlag 55 arretieren. Dabei entspannt sich der Übergangsabschnitt 22 und bewegt den proximalen Abschnitt 21 seitlich in Richtung Längsachse L. Zusätzlich oder alternativ zu einer Befestigung der Vorrichtung 10 und des Instruments 11 aneinander durch Aufnahme des Instruments 10 in der Vorrichtung 10 ist es beispielsweise möglich, die Vorrichtung 10 mit einem oder mehreren Klammern oder Clips mit elastisch nachgiebigen Abschnitten an dem Instrument 11 zu befestigen (nicht dargestellt), wobei die elastisch verformten Abschnitte die Vorrichtung 10 und/oder das Instrument 11 zwischen sich festklemmen.

Wenn das Instrument 11 in der Vorrichtung 10 angeordnet ist, schließt die Elektrode 15 oder der Elektrodenaufnahmeschaft 14, den Bereich 44 der Vorrichtung 10, in welchem die optische Faser 24 endet, gegenüber dem Bodenwandabschnitt 46 weitgehend, wie beispielhaft Figuren 4b, 4c und 4d veranschaulichen. Die Elektrode 15 und die Vorrichtung 10 begrenzend damit einen Kanalabschnitt 58, zwischen dem Ausgang der Leitung 28 an deren distalem Ende 32 und dem distalen Ende 29 der Vorrichtung 10. Der Kanalabschnitt 58 kann im Betrieb des Instruments 11 mit der Vorrichtung 10 über die Leitung 28 mit Spülmedium gespeist werden. Der Fluss Spülmedium streift die den Kanalabschnitt 58 begrenzende Unterseite 16a der Elektrodenaufnahmeschaft 14 und/oder der Elektrode 15 und kühlt diese. Damit die Elektrode 15 ausreichend Wärme abgibt, liegt die Oberseite 16b der Elektrodenaufnahmeschaft 14 auf der anderen Seite neben dem Kanalabschnitt 58 vorzugsweise frei. Zwischen der Oberseite 16b und der Umgebung der Vorrichtung 10 bzw. des Instruments 11 ist insbesondere kein Wandabschnitt der Vorrichtung 10 angeordnet.

Der Chirurg kann mit dem Instrument 11 bzw. der Vorrichtung 10 beispielsweise wie folgt arbeiten: Der Applikator 11 ist bevorzugt auch ohne die Vorrichtung 10 verwendbar. Bei der chirurgischen Anwendung des Applikators 11 führt der Anwender den Applikator 11 am Handgriffteil 12 haltend und wirkt dabei mittels des Applikators 11 auf das Gewebe ein. Beispielsweise kann der Chirurg mit dem Applikator 11 ohne damit verbundene Vorrichtung 10 den OP-Situs HF-chirurgisch öffnen und freilegen. In dieser Phase des medizinischen Eingriffs ist in der Regel noch keine Lichtanalyse erforderlich. Eine unnötige Verschmutzung der optischen Faser 24 kann somit in dieser Phase gänzlich vermieden werden. Hat der Chirurg die Stelle, an welcher der Präparationseingriff durchgeführt werden soll, derart vorbereitet, kann dieser oder ein Chirurgieassistent die Vorrichtung 10 und damit die optische Faser 24 zügig und lagerichtig mit dem Instrument 11 verbinden. Eine zuverlässige Lichtanalyse mittels der optischen Faser 24 ist nun möglich.

Ein die Vorrichtung 10 aufweisendes System kann dazu eingerichtet sein, dass bei verbundener Vorrichtung 10 mit dem Instrument 11 das Strömen des Spülmediums automatisch vor dem Speisen des Applikators 11 mit elektrischer Hochfrequenz(HF)-Energie eingeschaltet wird, um einer Verschmutzung durch Umspülen der optischen Faser 24 am distalen Ende von vornherein entgegenwirken zu können. Beispielsweise kann ein Gas als Spülmedium verwendet werden. Alternativ kann das System beispielsweise dazu eingerichtet sein, dass - unter der Voraussetzung, dass das Instrument 11 mit der Vorrichtung 10 verbunden ist - das Speisen der Elektrode 15 mit HF-Energie nur freigegeben ist, wenn die Leitung 28 mit Spülmedium gespeist wird. Der Spülmediumstrom bremst von Eingriffsort wegspritzende Tröpfen und/oder fliegenden Partikel ab und lenkt diese möglichst ab, so dass sie nicht auf den Lichteingang 31 der optischen Faser 24 treffen. Das System kann den Massen- oder Volumenstrom des Spülmediums bevorzugt auf einen Wert einstellen oder einen maximalen Wert beschränken, bei welchem das Zielgewebe oder die Zielstruktur, an welcher der Eingriff durchgeführt werden soll, nicht die chirurgische Präzision beeinträchtigend verdrängt wird, aber Partikel und/oder Tröpfchen zuverlässig abgebremst und/oder abgelenkt werden. Auch eine Gasembolie wird mittels Einstellung oder Beschränkung des maximalen Volumenstroms und Drucks durch das System vorzugsweise vermieden. In engen Körperhöhlen kann der Volumenstrom des Spülmediums zusätzlich Rauchgas und/oder flüssige Medien wie Blut oder geschmolzenes Fett verdrängen und so eine bessere Sicht auf den OP-Situs gewähren.

Der Chirurg kann die zusammengesetzte Gruppe aus Instrument 11 und Vorrichtung 10, welche den Handgriff 12 des Instruments 11 enthält, zum Arbeiten an der Vorrichtung 10 ergreifen. Fortan dient also die Vorrichtung 10 zumindest als ein Teil eines Griffstücks zum Handhaben der zusammengesetzten Gruppe. Nun wird die HF-Energieversorgung der Elektrode 15 wieder eingeschaltet und der Chirurg kann mit dem Instrument an dem OP-Situs weiterarbeiten. Dabei erzeugtes Licht, beispielsweise Funkenlicht, tritt in den Lichteingang 31 ein und wird mittels einer nicht gezeigten Analyseeinrichtung analysiert, um beispielsweise dem Chirurgen Informationen über das behandelte Gewebe zu geben.

Während Figuren 1a bis 4e ein Ausführungsbeispiel der Vorrichtung 10 zeigen, bei welchem die Lichtaufnahmeeinrichtung 24 schräg auf eine flache Seite 50b des Arbeitsabschnitts 15a der Elektrode 15 schaut, die flache Seite 50b dem Lichteingang 31 also schräg zugewandt ist, kann der Lichteingang 31 der Lichtaufnahmeeinrichtung 24 auch einer schmalen Seite (Schmalseite oder Kante) 50d eines spatelförmigen Arbeitsabschnitts 15a einer Elektrode 15 zugewandt sein. Eine solche relative Lage von Arbeitsabschnitt 15a der Elektrode 15 und Lichtaufnahmeeinrichtung 24 ist in Figur 5a anhand eines Beispiels gezeigt. Die Mittelachse MA des Lichtakzeptanzkegels 47 ist in einem spitzen Winkel zur Längsachse ELA der Elektrode 15 ausgerichtet.

Figur 5b zeigt ein anderes Beispiel, in welchem die Lichtaufnahmeeinrichtung 24 in einer solchen Lage relativ zu der Elektrode 15 angeordnet ist, dass die Mittelachse MA des Lichtakzeptanzkegels 47 senkrecht zu der Längsachse ELA der Elektrode 15 verläuft.

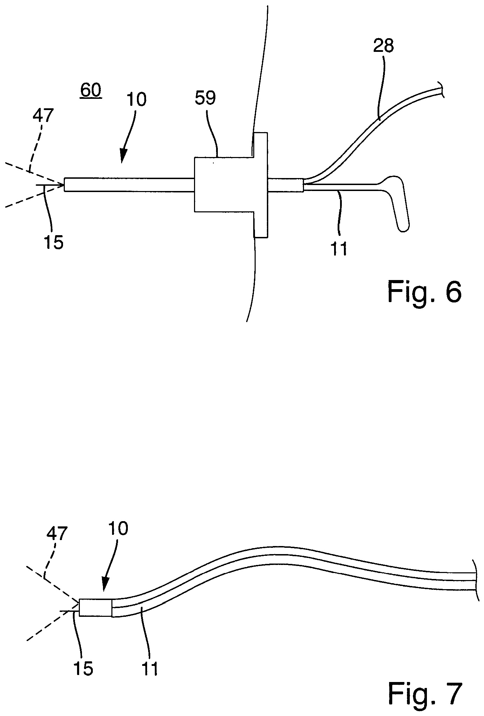

Figur 6 zeigt ein Ausführungsbeispiel einer Vorrichtung 10 zur Befestigung der Lichtaufnahmeeinrichtung 24, welche einen Lichtakzeptanzkegel 47 festlegt, zur Lichtanalyse an einem Instrument 11 für die Laparoskopie, welches durch einen Trokar 59 in die Bauchhöhle 60 eingeführt ist. Das Instrument 11 weist eine Elektrode 15 auf, welche mit elektrischer HF-Energie gespeist werden kann. Der Trokar 59 stellt einen Zugang für das Instrument 11 mit verbundener Vorrichtung 10 bereit, wobei die Vorrichtung 10 und das Instrument 11 dazu eingerichtet sind, dass der Zugang bei eingeführter Vorrichtung 10 und Instrument 11 um die Vorrichtung 10 und das Instrument 11 so dicht bleibt, so dass durch den Trokar 59 kein Gas unerwünscht aus der Bauchhöhle 60 entweicht. Die geometrische Außenkontur ist bevorzugt rund ausgeführt, damit der Zugang durch den Trokar 59 entsprechend dicht ist. Das Instrument 11 und die optische Faser 24 können parallel in einem Rohr, z.B. einem Hüllrohr geführt werden, um eine mechanische Beschädigung zu vermeiden. Die Vorrichtung 10 kann beispielsweise ein von dem Anwender des Instruments 11 an dem Instrument 11 befestigbarer, und bevorzugt von dem Instrument 11 lösbarer, Adapter sein oder an dem Instrument 11 selbst ausgebildet sein. Beispielsweise ist die Vorrichtung 10 eine Öffnung oder ein Kanal am Instrument 11, in welche eine optische Faser 24 bei Bedarf eingeschoben oder eingeclipst werden kann.

Figur 7 zeigt ein Ausführungsbeispiel einer Vorrichtung 10 zur Befestigung einer Lichtaufnahmeeinrichtung 24 zur Lichtanalyse an einem Instrument 11 für die endoskopische Chirurgie. Bei dem Instrument 11 kann es sich beispielsweise um eine flexible Sonde mit einer Elektrode 15 handeln, welche das Schneiden und/oder Koagulieren mittels einer mit HF-Energie gespeisten Elektrode 15 erlaubt, deren Lichterscheinungen mittels der Lichtaufnahmeeinrichtung 24 untersucht werden können. Das Instrument 11 kann beispielsweise eine Argon-Plasma-Koagulationssonde sein. Das Instrument 11 kann alternativ oder zusätzlich dazu eingerichtet sein, eine dielektrische Barriereentladung zu nutzen.

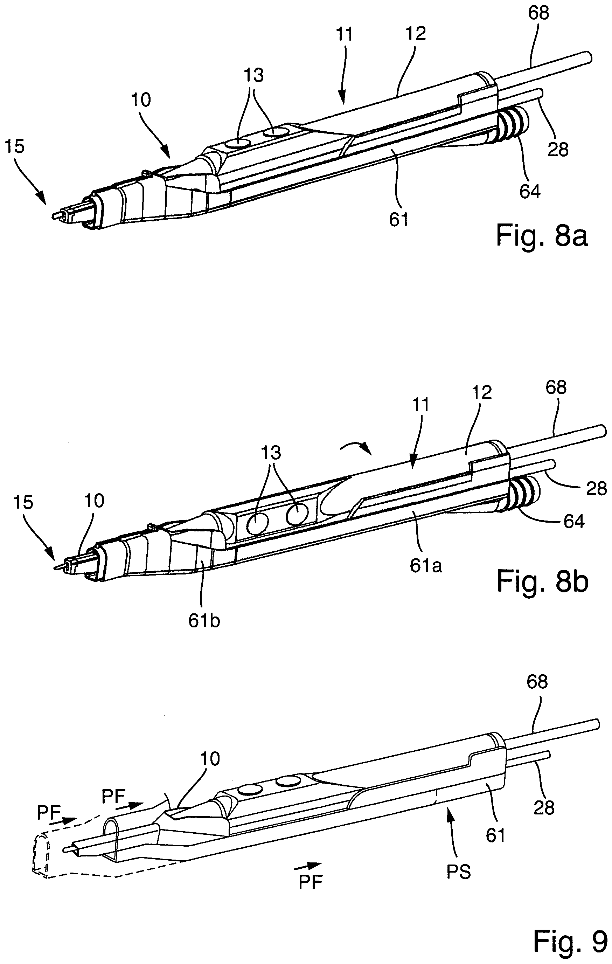

Die Figuren 8a bis 10b zeigen ein Beispiel einer Ausführungsform der Vorrichtung 10, welche dazu eingerichtet ist, optional mit einer Absaugeinheit 61 verbunden zu werden. Die Absaugeinheit 61 kann von dem Chirurgen oder seinem Assistenten vorzugsweise während der Operation mit der Vorrichtung 10 verbunden werden. Wie in Figur 9 mit den Pfeilen PF gezeigt, ist die Absaugeinheit 61 bevorzugt auf die Vorrichtung 10 schiebbar, beispielsweise wenn die Vorrichtung 10 mit dem Instrument 11 verbunden ist. Die Absaugeinheit 61 ist vorzugsweise werkzeuglos mit der Vorrichtung 10 verbindbar und zerstörungsfrei von der Vorrichtung 10 lösbar. Wie mit dem Pfeil PS angedeutet, kann die Absaugeinheit 61 einen proximalen Abschnitt 61a aufweisen, welcher flexibel elastisch mit dem distalen Abschnitt 61b verbunden ist und welcher zum Verbinden der Absaugeinheit und der Vorrichtung zunächst weggebogen werden muss und, nach dem Ineinanderschieben von Absaugeinheit 61 und Vorrichtung 10 und elastischem Entspannen in die Ausgangslage, die Vorrichtung 10 zusammen mit dem distalen Abschnitt 61b formschlüssig in der Absaugeinheit 61 fixiert. Dies entspricht der bevorzugten Befestigung der Vorrichtung 10 am Handgriff 12 wie vorstehend beschrieben. Es ist alternativ oder zusätzlich beispielsweise möglich, die Absaugeinheit 61 mit einem oder mehreren Klammern oder Clips mit elastisch nachgiebigen Abschnitten an der Vorrichtung 10 und/oder dem Instrument 11 zu befestigen (nicht dargestellt), wobei die elastisch verformten Abschnitte die Vorrichtung 10 und/oder das Instrument 11 zwischen sich festklemmen.

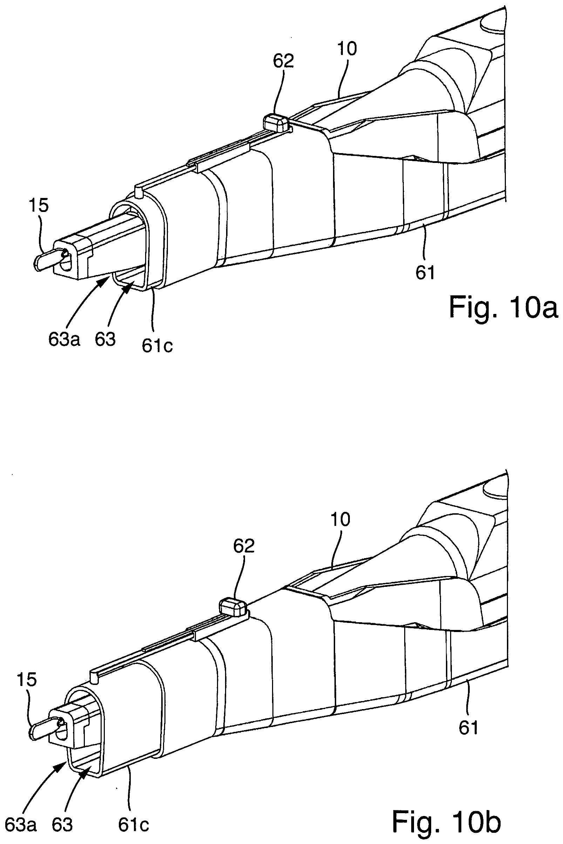

Bevorzugt weist die Absaugeinheit 61 einen Fortsatzabschnitt 61c auf, der teleskopartig in dem distalen Abschnitt 61b geführt und aus einer Proximallage (in Figur 10a gezeigt) durch Vorschieben eines Schieberabschnitts 62 bei Bedarf in distale Richtung in eine Distallage (in Figur 10b gezeigt) vorschiebbar ist.

Die Absaugeinheit 61 kann am distalen Ende transparent sein, um eine verbesserte Sicht auf den OP-Situs zu gewähren. Beispielsweise besteht die Absaugeinheit 61 oder, falls vorhanden, zumindest der Fortsatzabschnitt 61c aus transparentem Kunststoff. In bevorzugten Ausführungsformen ist sowohl die Absaugeinheit 61 als auch die Vorrichtung 10 an deren distalen Enden transparent.

Alternativ zu der vorstehend beschriebenen Ausführungsform kann die Absaugeinheit 61 auch ein untrennbarer Bestandteil der Vorrichtung 10 sein.

Die Absaugeinheit 61 dient dazu, beim HFchirurgischen Einsatz entstehenden Rauch vom OP-Situs abzusaugen. Die Absaugeinheit 61 weist einen Absaugkanal 63 auf, welcher sich im dargestellten Ausführungsbeispiel durch den distalen Abschnitt 61b und den Fortsatzabschnitt 61c bis zu einer Absaugöffnung 63a am distalen Ende der Absaugeinheit 61 erstreckt. Die Vorrichtung 10, welche den Spülkanal 58 begrenzt und die optische Faser 24 enthält, erstreckt sich durch die Absaugeinheit 61 und im dargestellten Ausführungsbeispiel durch den Absaugkanal 63 und insbesondere durch die Absaugöffnung 63a. Während die elektrische Leitung 68 zur Speisung der Elektrode 15 mit HF-Leistung (HF-Versorgungleitung), die Leitung für das Spülmedium und die Absaugleitung 64 in Figur 8a bis 8b nebeneinander an den Handgriff 12, die Vorrichtung 10 bzw. die Absaugeinheit 61 herangeführt dargestellt sind, kann beispielsweise die Leitung 28 für das Spülmedium, die vorzugsweise auch die optische Faser 24 enthält, proximal des Handgriffs 12 innerhalb der Absaugleitung 64 angeordnet sein.

Die Absaugöffnung 63a ist bei diesem Ausführungsbeispiel gegenüber der Mündung des Spülkanals 58a bevorzugt in jeder Stellung des Fortsatzabschnitts 61c nach proximal zurückversetzt. In Figur 4d ist die Mündung des Spülkanals 58a beispielsweise die Mündung des Kanalabschnitts 58 am distalen Ende der Vorrichtung 10. Bevorzugt ist die Öffnungsfläche der Mündung 58a des Spülkanals 58 am distalen Ende 29 der Vorrichtung 10 und/oder die Öffnungsfläche der Öffnung 28a der Leitung 28 für das Spülmedium an deren distalem Ende 32 kleiner als die freie Öffnungsfläche der Absaugöffnung 63a des Absaugkanals. Auf diese Weise kann eine vergleichsweise hohe Strömungsgeschwindigkeit in der Mündung 58a des Spülkanals 58 und/oder der Öffnung 28a der Leitung 28 erreicht werden. Dennoch kann das System zur Vermeidung eines Aufblasens oder Aufweitens von Gewebe derart eingerichtet oder eingestellt sein, dass der Absaugvolumenstrom (z.B. 50 - 120 l/min) in den Absaugkanal um beispielsweise wenigstens das zehnfache größer ist als der Spülvolumenstrom (z.B. etwa 1 l/min) aus dem Spülkanal 58. Dies ist insbesondere dann von Vorteil, wenn das System dazu eingerichtet oder eingestellt ist, dass gleichzeitig mit Spülmedium gespült und mittels der Absaugeinheit 61 abgesaugt wird. Die freie Öffnungsfläche der Absaugöffnung 63a des Absaugkanals 63 umgibt die Elektrode 15 und den Teil der Vorrichtung 10 am distalen Ende 29, in dem der Spülkanal 58 und die Lichtaufnahmeeinrichtung 24 angeordnet sind, die sich durch die Absaugöffnung 63a des Absaugkanals 63 erstrecken.

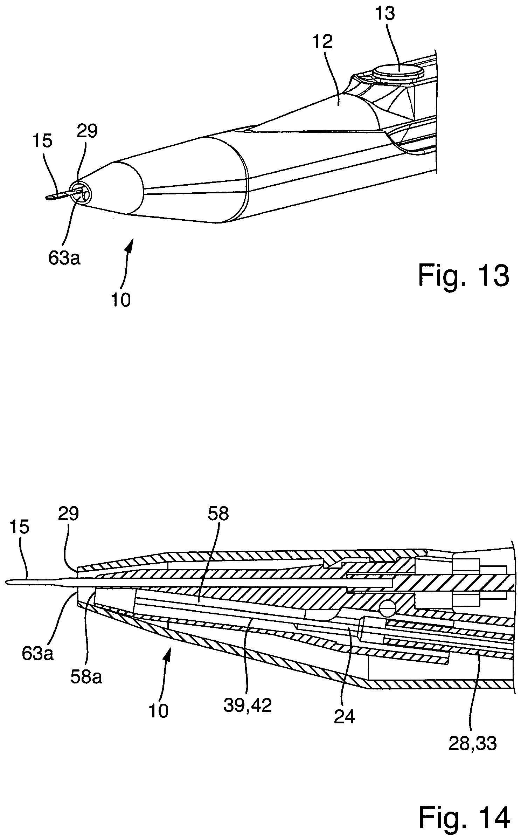

Bei einem anderen Ausführungsbeispiel kann die Absaugöffnung 63a näher am distalen Ende der Elektrode 15 angeordnet sein als die Mündung 58a des Spülkanals 58. Ein solches Ausführungsbeispiel ist in den Figuren 13 und 14 veranschaulicht. Bei diesem Ausführungsbeispiel können andere Volumenströme für das Spülmedium bzw. die Absaugung verwendet werden. Beispielsweise kann der Volumenstrom der Absaugung etwa 6-8 Mal größer sein als der Volumenstrom des Spülmediums bzw. Spülgases. Bei einem Ausführungsbeispiel kann der Absaugvolumenstrom 50 l/min. betragen und der Spülvolumenstrom des Spülmediums kann etwa 7,0 l/min. betragen.

Alle Literangaben bei den Volumenströmen beziehen sich auf das Normvolumen (Standard- oder Normliter) des Fluids.

Unabhängig davon, ob die Vorrichtung 10 mit einer Absaugeinheit 61 verbindbar ist oder ob die Absaugeinheit 61 einen untrennbaren Bestandteil der Vorrichtung 10 bildet, kann die Vorrichtung 10 bei einigen oder allen Ausführungsbeispielen eine diskrete Auswahl an möglichen Lagen der Lichtaufnahmeeinrichtung 24 relativ zu dem Applikator 11 festlegen. Wenn der Applikator 11 eine diskrete Auswahl an möglichen Lagen der Elektrode 15 relativ zu dem Handgriff 12 festlegt, legt die Vorrichtung 10 vermittelt über den Handgriff 12 eine diskrete Anzahl möglicher Lagen der Lichtaufnahmeeinrichtung 24 relativ zu der Elektrode 15 fest. Alle Lagen der Auswahl zeichnen sich dadurch aus, dass sichergestellt ist (wenn Vorrichtung 10, Instrument 11 und Lichtaufnahmeeinrichtung 24 für einander bestimmt sind), dass eine ausreichende Lichtmenge der Lichterscheinung, hervorgerufen durch das Instrument 11, von der Lichtaufnahmeeinrichtung 24 aufnehmbar ist. Die Lage (0°-Lage) gezeigt in Figur 8a unterscheidet sich von der Lage (90°-Lage) gemäß Figur 8b durch eine andere Drehorientierung (um 90° gedreht) der Vorrichtung 10 und damit der Lichtaufnahmeeinheit 24 um die Längsachse ELA der Elektrode 15. Die diskreten Lagen können beispielsweise durch Formschluss in Drehrichtung um die Längsachse ELA der Elektrode 15 mit Strukturen 56, 57 wie in Figur 4e veranschaulicht festgelegt sein. Das Instrument 11, dargestellt in den Figuren 8a und 8b, ist vorzugsweise so aufgebaut wie das Instrument 11 gemäß Figuren 4a bis 4d. Nur die Orientierung der Elektrode 15 ist derart, dass in der 0°-Lage die Lichtaufnahmeeinrichtung 24 auf eine schmale Seite 50d der Elektrode 15 schaut. In der 90°-Lage schaut die Lichtaufnahmeeinrichtung 24 entsprechend auf die Flachseite 50b der spatelförmigen Elektrode 15. Der Chirurg kann somit während der Operation auswählen, ob die Lichtaufnahmeeinrichtung 24 direkt auf eine Kante 50d, 50e der spaltelförmigen Elektrode 15 schauen soll (Figur 8a) oder ob die Lichtaufnahmeeinrichtung 24 Licht von beiden Kanten 50d, 50e etwa gleich erfassen können soll (Figur 8b). Letzteres kann vorteilhaft sein, wenn der Chirurg beide Kanten 50d, 50e zum Arbeiten nutzen möchte. Ersteres kann vorteilhaft sein, wenn der Chirurg nur die Kante 50d zum Arbeiten möchte, auf welche die Lichtaufnahmeeinrichtung 24 ausgerichtet ist, da diese dann von der Kante 50d mehr Licht empfangen kann als von der gegenüberliegenden Kante 50c.

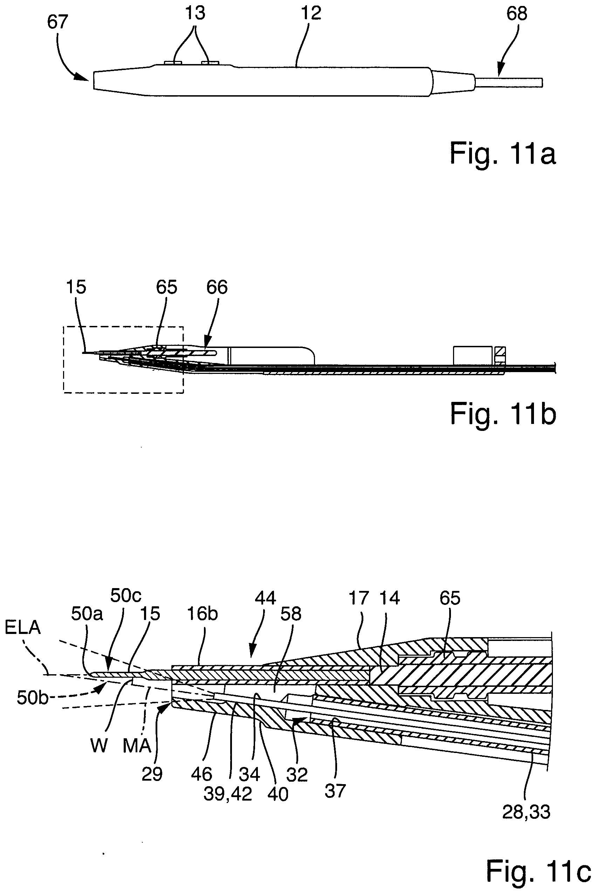

Für das in Figuren 11a bis 11c veranschaulichte Ausführungsbeispiel der Vorrichtung 10, können die Erläuterungen zu der Ausführungsform gemäß Figuren 4a bis 4d herangezogen werden, wobei sich die Ausführungsformen dadurch voneinander unterscheiden, dass die Vorrichtung 10 gemäß Figuren 11a bis 11c eine Elektrode 15 aufweist. Die Elektrode 15 wird von der Vorrichtung 10 getragen (s. die Längsschnittdarstellungen in den Figuren 11b und 11c) ohne, dass die Vorrichtung 10 mit dem Handgriff 12 verbunden sein muss. Die Vorrichtung 10 legt insbesondere die Lage der Lichtaufnahmeeinrichtung 24 relativ zu der Elektrode 15 bis auf beispielsweise solche fest, in denen die Lichtaufnahmeeinrichtung 24 auf eine Flachseite 50b oder 50d der Elektrode 15 schaut - oder alternativ bis auf solche, in denen die Lichtaufnahmeeinrichtung 24 auf eine Kante 50d, 50e der Elektrode 15 schaut. Insbesondere kann die Vorrichtung 10 eine einzige mögliche Lage der Elektrode 15 relativ zu der Lichtaufnahmeeinrichtung 24 festlegen. Der Arbeitsabschnitt 15a der Elektrode 15 ist in einem Elektrodenaufnahmeschaft 14 aufgenommen, welcher in einer Aufnahmeausnehmung 35 aufgenommen ist. Über ein Kopplungselement 65 kann die Lage des Elektrodenaufnahmeschaftes 14 und damit der Elektrode 15 relativ zu der Vorrichtung 10 und damit zur optischen Faser 24 fixiert sein. Der Elektrodenaufnahmeschaft 14, der ein ein- oder mehrteiliges Element sein kann, weist kann eine Formschlussstruktur 66 zum mechanischen und elektrischen Koppeln des Handgriffs 12 mit dem Elektrodenaufnahmeschaft 14 aufweisen.

Der Handgriff 12 dazu ist in Figur 11a in Seitenansicht als gesonderte Instrumentenkomponente dargestellt. Das Zusammensetzen der Vorrichtung 10 und des Handgriffs 12 kann, wie vorstehend beschrieben, während der Operation erfolgen, mit dem Unterschied, dass ein arbeitsfähiges Instrument 11 aus Handgriff 12 und Elektrode 15 beim Zusammensetzen entsteht. Dabei wird der Elektrodenaufnahmeschaft 14, in welchem die Elektrode 15 aufgenommen ist, in eine Aufnahme 67 des Handgriffs 12 geschoben und beispielsweise mittels der Formschlussstruktur 66 fixiert. Dabei wird der Elektrodenaufnahmeschaft 14 vorzugsweise elektrisch mit den HF-Versorgungsleitungen 68 zum Handgriff 12 verbunden. Die Elektrode 15 kann dann über den Elektrodenaufnahmeschaft 14 mit elektrischer Energie versorgt werden. Ausführungsbeispiele der Ausführungsform gemäß Figuren 11a bis 11c können zulassen, dass der Handgriff 12 und die Vorrichtung 10 entsprechend Figuren 8a und 8b in beispielsweise 90° drehversetzten Lagen miteinander kombinierbar sind, ohne die relative Lage der Lichtaufnahmeeinrichtung 24 und der Elektrode **15 (z.B.** Lichtaufnahmeeinrichtung 24 schaut auf Flachseite 50b der Elektrode 15) zu verändern.

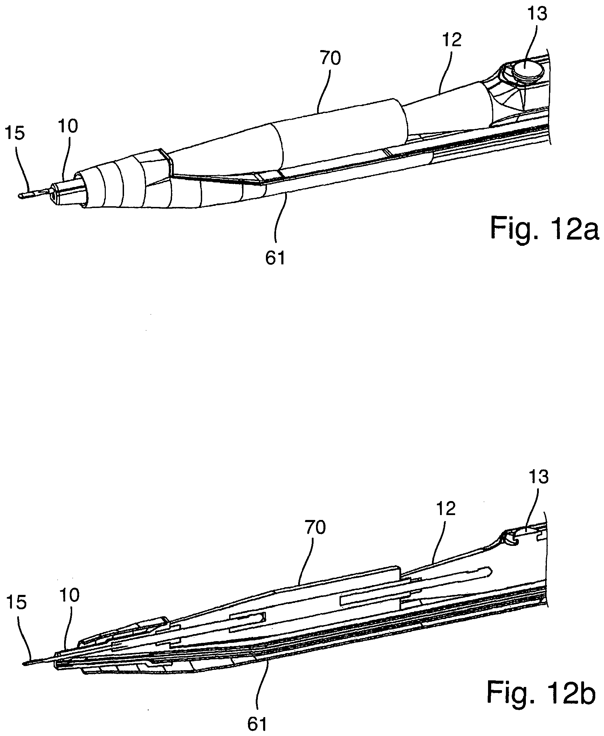

Die Figuren 12a bis 12b zeigen einen Handgriff 12, der mit einer erfindungsgemäßen Vorrichtung 10 gekoppelt ist. Zwischen dem Handgriff 12 und dem distalen Ende der Vorrichtung 10 ist, in der Vorrichtung 10 aufgenommen, ein Verlängerungselement 70 angeordnet. Das Verlängerungselement 70 koppelt den Handgriff 12 mit der Vorrichtung 10 mechanisch und die HF-Versorgungsleitung 68 mit der Elektrode 15 über ein Leitungsstück 71 elektrisch. Die Elektrode 15 kann von dem Verlängerungselement 70 oder der Vorrichtung 10 getragen werden. Für Beispiele von Ausführungsformen, in denen die Elektrode 15 von dem Verlängerungselement 71 getragen wird, können die obenstehenden Erläuterungen zu Ausführungsformen herangezogen werden, in denen die Elektrode 15 von einer Instrumentenkomponente, z.B. dem Handgriff 12, des Instruments 11 getragen wird. Für Beispiele von Ausführungsformen, in denen die Elektrode 15 von der Vorrichtung 10 getragen wird, können die obenstehenden Erläuterungen zu Ausführungsformen herangezogen werden, in denen die Elektrode 15 von der Vorrichtung 10 getragen wird.

Es wird eine erfindungsgemäße Vorrichtung 10 zur Befestigung, durch den Anwender eines chirurgischen Instruments 11, einer Lichtaufnahmeeinrichtung 24 zur Lichtanalyse an dem Instrument 11 oder einer Instrumentenkomponente 12 des Instruments 11 angegeben. Die Vorrichtung 10 ist bevorzugt dazu eingerichtet, die Lichtaufnahmeeinrichtung 24 lösbar an dem Instrument 11 oder der Instrumentenkomponente 12 zu fixieren. Bevorzugt ist die Vorrichtung 10 dazu ausgelegt, dass die Vorrichtung 10 wiederholt gelöst und wiederholt genutzt werden kann. Bei dem Instrument kann es sich in einfachen Fällen um einen Handgriff 12 mit einer Elektrode 15 handeln. Ausführungsformen der Vorrichtung 10 weisen sowohl eine Lichtaufnahmeeinrichtung 24 als auch eine relativ dazu fixierte Elektrode 15 auf, wobei die Vorrichtung 10 an dem Handgriff 12 fixierbar ist. Es sind Ausführungsformen möglich, in denen die Elektrode 15 nicht von der Vorrichtung 10 getragen wird, sondern von dem Instrument 11. Es werden jedoch Ausführungsformen bevorzugt, in denen die Elektrode 15 von der Vorrichtung 10 zerstörungsfrei lösbares oder in der Vorrichtung 10 festverbautes Teil der Vorrichtung 10 ist. Es werden Ausführungsformen bevorzugt, in denen die Vorrichtung 10 von dem chirurgischen Anwender oder seinem Assistenten an dem Instrument 11 oder der Instrumentenkomponente 12 befestigbar (und bevorzugt wieder lösbar) ist und einen Adapter zum Befestigen einer Lichtaufnahmeeinrichtung 24 an dem Instrument 11 oder der Instrumentenkomponente 12 bildet. In anderen Ausführungsformen ist die Vorrichtung 10 an dem Instrument 11 ausgebildet, beispielsweise ein Kanal in dem Instrument 11, in welchen eine optische Faser 24 eingeschoben werden kann. Bevorzugt ist mittels der Vorrichtung 10 eine Rotation oder ein Verschieben der Lichtaufnahmeeinrichtung 24 relativ zu der Elektrode 15 so eingeschränkt, dass das distale Ende der Elektrode 15 und/oder die Lichterscheinungen, hervorgerufen durch Beaufschlagung der Elektrode 15 mit elektrischer Hochfrequenzenergie bei der Anwendung des Instruments 11 stets in dem Lichtakzeptanzkegel 47 der Lichtaufnahmeeinrichtung 24 bleiben. Bevorzugt ist die Form einer ersten Aufnahme 25 der Vorrichtung 10 auf die Form des Instruments 11 abgestimmt, um die relative Lage der Lichtaufnahmeeinrichtung 24 relativ zu der Elektrode 15 bis auf solche relativen Lagen vorzugeben, so dass die Spitze oder das distale Ende 50 der Elektrode 15 und/oder die Lichterscheinungen, hervorgerufen durch Beaufschlagung der Elektrode 15 mit elektrischer Hochfrequenzenergie bei der Anwendung des Instruments 11 in jeder verbleibenden Lage im Lichtakzeptanzkegel 47 der Lichtaufnahmeeinrichtung 24 sind.

**Bezugszeichenliste:**

| | |
|---|---|
| 10 | Vorrichtung |
| 11 | Instrument/Applikator |
| 12 | Handgriff |
| 13 | Bedienelemente |
| 14 | Elektrodenaufnahmeschaft |
| 15 | Elektrode |
| 15a | Arbeitsabschnitt |
| 16a | Unterseite der Elektrodeschaftaufnahme |
| 16b | Oberseite der Elektrodeschaftaufnahme |
| 17 | Distaler Abschnitt |
| 18 | Proximaler Endabschnitt/Halbschalenform |
| 19 | Längsseite |
| 20 | Oberseite |
| 21 | Proximaler Abschnitt |
| 22 | Übergangsabschnitt |
| 24 | Lichtaufnahmeeinrichtung/optische Faser |
| 25 | Erste Aufnahme/erster Aufnahmekanal |
| 26 | Formschlussabschnitt |
| 27 | Zweite Aufnahme |
| 28 | Leitung |
| 28a | Öffnung (Mündung) der Leitung |
| 29 | Distales Ende der Vorrichtung |
| 30 | Wandfläche |
| 31 | Lichteingang |
| 32 | Distales Ende der Leitung |
| 33 | Leitungsabschnitt |
| 34 | Endabschnitt der optischen Faser |
| 35 | Aufnahmebohrung |
| 36 | Halteelemente |
| 37 | Innenwandfläche |
| 38 | Distales Ende der optischen Faser |
| 39 | Abstützabschnitt |
| 40 | Aufnahmewand |
| 41 | Biegung |
| 42 | Aufnahmebereich |
| 44 | Bereich |
| 45a,b | Seitenwandabschnitt |
| 46 | Bodenwandabschnitt |
| 47 | Lichtakzeptanzkegel |
| 50a | Spitze der Elektrode |
| 50b | Flache Seite (Flachseite oder Spatelseite) der Elektrode |
| 50c | Flache Seite (Flachseite oder Spatelseite) der Elektrode |
| 50d | Schmale Seite (Schmalseite oder Kante) der Elektrode |
| 50e | Schmale Seite (Schmalseite oder Kante) der Elektrode |
| 51 | Erster Anschlag |
| 52 | Wandabschnitt |
| 53 | Erster Gegenanschlag |
| 54 | Zweiter Anschlag |
| 55 | Zweiter Gegenanschlag |
| 56 | Ausrichtstruktur |
| 57 | Gegenausrichtstruktur |
| 58 | Kanalabschnitt |
| 58a | Mündung des Kanalabschnitts/Spülkanals |
| 59 | Trokar |
| 60 | Bauchhöhle |
| 61 | Absaugeinheit |
| 61a | Proximaler Abschnitt |
| 61b | Distaler Abschnitt |
| 61c | Fortsatzabschnitt |
| 62 | Schieberabschnitt |
| 63 | Absaugkanal |
| 63a | Absaugöffnung |
| 64 | Absaugleitung |
| 65 | Kopplungselement |
| 66 | Formschlussstruktur |
| 67 | Aufnahme |
| 68 | Versorgungsleitung |
| 70 | Verlängerungselement |
| 71 | Leitungsstück |
| B | Breite |
| L | Längsachse |
| OA | Optische Achse |
| MA | Mittelachse |
| MAV | Mittelachse des distalen Abschnitts |
| ELA | Elektrodenlängsachse |
| LK | Lichtaktzeptanzkegel |
| W1 | Winkel |
| W2 | Winkel zwischen Längsachse optische Faser und erster Aufnahme/Elektrode |
| P | Pfeil |
| PP | Pfeil |
| PF | Pfeil |
| PS | Pfeil |
| R | Axialrichtung |

## Patentansprüche

1. Vorrichtung (10), die dazu eingerichtet ist, während einer Operation eine Lichtaufnahmeeinrichtung (24) zur Lichtanalyse an einem chirurgischen Instrument (11) oder an einer Instrumentenkomponente (12) des chirurgischen Instruments (11) durch den chirurgischen Anwender des chirurgischen Instruments (11) oder seinen Assistenten zu befestigen, **dadurch gekennzeichnet, dass** die Vorrichtung (10) außerdem dazu eingerichtet ist, Licht innerhalb eines Lichtakzeptanzkegels (47) an einem Lichteingang (31) aufzunehmen und zu einer Analysevorrichtung weiterzuleiten, mittels welcher das Licht analysiert werden kann, wobei das Licht bei der Verwendung des chirurgischen Instruments (11) erzeugt wird und wobei sich eine Spitze oder ein distales Ende (50a) einer Elektrode (15) oder des Arbeitsabschnitts (15a) innerhalb des Lichtakzeptanzkegels (47) befindet, wenn die Vorrichtung (10) an einem Instrument (11) befestigt ist.

2. Vorrichtung (10) nach Anspruch 1, wobei die Vorrichtung (10) eine vorbestimmte Auswahl an einer einzigen oder mehreren räumlichen Lagen der befestigten Lichtaufnahmeeinrichtung (24) relativ zu der Instrumentenkomponente (12) und/oder dem Instrument (11) festlegt.

3. Vorrichtung (10) nach einem der vorstehenden Ansprüche für ein Instrument (11), welches eine mit elektrischer HF-Energie beaufschlagbare Elektrode (15) aufweist, wobei die Vorrichtung (10) eine vorbestimmte Auswahl an einer einzigen oder mehreren räumlichen Lagen der befestigten Lichtaufnahmeeinrichtung (24) relativ zu der Elektrode (15) festlegt.

4. Vorrichtung (10) nach einem der vorstehenden Ansprüche, wobei die Vorrichtung (10) eine Elektrode (15) trägt und eine vorbestimmte Auswahl an einer einzigen oder mehreren räumlichen Lagen der Lichtaufnahmeeinrichtung (24) relativ zu der Elektrode (15) festlegt.

5. Vorrichtung (10) nach einem der vorstehenden Ansprüche mit einer Aufnahme (25), welche die Beweglichkeit des Instruments in der Aufnahme (25) auf eine Beweglichkeit in einer Axialrichtung (L) einschränkt und welche dazu eingerichtet ist, das Instrument (11) in der Axialrichtung (L) aufzunehmen, und mit einer Einrichtung (17, 51, 53, 21, 54, 55) zum anschließenden Festlegen oder Einschränken der axialen Position des Instruments (11) in der Aufnahme (25).

6. Vorrichtung (10) nach Anspruch 5, wobei die Einrichtung (17, 51, 53, 21, 54, 55) zum anschließenden Festlegen oder Einschränken der axialen Position einen quer relativ zur der Axialrichtung (L) beweglichen Abschnitt (21, 22) aufweist, welcher dazu eingerichtet ist, nach dem Anordnen des Instruments (11) in der Aufnahme (25) durch seitliche Bewegung des Abschnitts (21) in Eingriff mit dem Instrument (11) gebracht zu werden, um die axiale Position des Instruments (11) in der Aufnahme (25) festzulegen oder einzuschränken.

7. Vorrichtung (10) nach Anspruch 5, wobei der Abschnitt (21, 22) flexibel befestigt ist.

8. Vorrichtung (10) nach einem der vorstehenden Ansprüche, wobei die Vorrichtung (10) einen Spülkanal (28, 58) aufweist, wobei der Spülkanal (28, 58) dazu eingerichtet ist, ein Spülmedium seitlich an dem Lichteingang (31) der Lichtaufnahmeeinrichtung (24) vorbei auszugeben.

9. Vorrichtung (10) nach einem der vorstehenden Ansprüche, wobei die Vorrichtung (10) die räumliche Lage des Spülkanals relativ zu der Lichtaufnahmeeinrichtung (24) festlegt.

10. Vorrichtung (10) nach einem der vorstehenden Ansprüche, wobei der Lichteingang (31) der Lichtaufnahmeeinrichtung (24) in der Vorrichtung (10) angeordnet ist.

11. Vorrichtung (10) nach einem der Ansprüche 8 bis 10, wobei der Lichteingang (31) innerhalb des Spülkanals (58) angeordnet ist.

12. Vorrichtung (10) nach einem der vorstehenden Ansprüche, wobei die Lichtaufnahmeeinrichtung (24) vollständig oder teilweise innerhalb des Spülkanals (28) verläuft.

13. Vorrichtung (10) nach einem der vorstehenden Ansprüche, wobei, begrenzt einerseits durch eine Wand (45a, 45b, 46) der Vorrichtung (10) und begrenzt andererseits durch eine erste Längsseite (16a) einer Elektrode (15), beispielsweise eines Elektrodenschafts (15a), der Vorrichtung (10), ein Kanalabschnitt (58) gebildet ist, welcher Kanalabschnitt (58) von dem Spülmedium durchströmbar ist, wobei eine der ersten Längsseite (16a) gegenüberliegende zweite Längsseite (16b) der Elektrode (15), beispielsweise eine zweite Längsseite (16b) des Elektrodenschafts (15a), entlang des Kanalabschnitts (58) wenigstens abschnittsweise freiliegt.

14. Vorrichtung (10) nach einem der vorstehenden Ansprüche, wobei die optische Achse (OA) der Lichtaufnahmeeinrichtung (24) und die Elektrode (15) einen spitzen Winkel (W) einschließen.

15. Verfahren zum Befestigen einer Lichtaufnahmeeinrichtung (24) an einem chirurgischen Instrument (11) oder einer Instrumentenkomponente (12) des chirurgischen Instruments (11) mittels einer Vorrichtung (10) nach einem der vorstehenden Ansprüche durch den Chirurgen oder seinen Assistenten während einer Operation.

## Claims

1. Apparatus (10) configured for attachment of a light receiving device (24) for light analysis on an instrument (11) or on an instrument component (12) of the surgical instrument (11) by the surgical user of the surgical instrument (11) or his assistant during an operation, **characterized in that**, the apparatus (10) is further configured to capture light within a light acceptance angle (47) at a light inlet (31) and to transmit it to an analysis device by means of which the light can be analyzed, wherein the light is created during use of the surgical instrument (11) and wherein a tip or a distal end (50a) of an electrode (15) or of a working section (15a) is located inside the light acceptance angle (47), if the apparatus (10) is attached to an instrument (11)..

2. Apparatus (10) according to claim 1, wherein the apparatus (10) defines a predefined variety of one single or multiple spatial positions of the attached light receiving device (24) relative to the instrument component (12) and/or the instrument (11).

3. Apparatus (10) according to any of the preceding claims for an instrument (11) that comprises an electrode (15) that can be supplied with electrical RF energy, wherein the apparatus (10) defines a predefined variety of one single or multiple spatial positions of the attached light receiving device (24) relative to the electrode (15).

4. Apparatus (10) according to any of the preceding claims, wherein the apparatus (10) supports an electrode (15) and defines a predefined variety of one single or multiple spatial positions of the light receiving device (24) relative to the electrode (15).

5. Apparatus (10) according to any of the preceding claims, having a mount (25) that limits the movability of the instrument in the mount (25), except for a movability in an axial direction (L) and that is configured to hold the instrument (11) in axial direction (L) and having a device (17, 51, 53, 21, 54, 55) for subsequent definition or limitation of the axial position of the instrument (11) in the mount (25).

6. Apparatus (10) according to claim 5, wherein the device (17, 51, 53, 21, 54, 55) for subsequent definition or limitation of the axial position comprises a section (21, 22) movable transverse with regard to the axial direction (L) that is configured to engage the instrument (11) by means of a lateral movement of the section (21) after arrangement of the instrument (11) in the mount (25) in order to define or limit the axial position of the instrument (11) in the mount (25).

7. Apparatus (10) according to claim 5, wherein the section (21, 22) is attached in a flexible manner.

8. Apparatus (10) according to any of the preceding claims, wherein the apparatus (10) comprises a flushing channel (28, 58), wherein the flushing channel (28, 58) is configured to output a flushing medium passing the light inlet (31) of the light receiving device (24) laterally.

9. Apparatus (10) according to any of the preceding claims, wherein the apparatus (10) defines the spatial position of the flushing channel relative to the light receiving device (24).

10. Apparatus (10) according to any of the preceding claims, wherein the light inlet (31) of the light receiving device (24) is arranged inside the apparatus (10).

11. Apparatus (10) according to any of the preceding claims 8-10, wherein the light inlet (31) is arranged inside the flushing channel (58).

12. Apparatus (10) according to any of the preceding claims, wherein the light receiving device (24) extends completely or partly inside the flushing channel (28).

13. Apparatus (10) according to any of the preceding claims, wherein a channel section (58) is formed limited on one side by a wall (45a, 45b, 46) of the apparatus (10) and limited on the other side by a first longitudinal side (16a) of an electrode (15) of the apparatus (10), e.g. an electrode holding shank (14), wherein the channel section (58) can be flushed with the flushing medium, wherein a second longitudinal side (16b) of electrode (15) opposite the first longitudinal side (16a), e.g. a second longitudinal side (16b) of electrode holding shank (14), is exposed at least in sections along the channel section (58).

14. Apparatus (10) according to any of the preceding claims, wherein the optical axis (OA) of the light receiving device (24) and the electrode (15) include an acute angle (W).

15. Method for attaching a light receiving device (24) on a surgical instrument (11) or an instrument component (12) of the surgical instrument (11) by means of a device (10) according to any of the preceding claims by a surgeon or his assistant during a surgery.

## Revendications

1. Dispositif (10) qui est conçu pour fixer, pendant une opération, un dispositif de réception de lumière (24), destiné à l'analyse de la lumière, sur un instrument chirurgical (11) ou sur un composant d'instrument (12) de l'instrument chirurgical (11), par l'utilisateur chirurgien de l'instrument chirurgical (11) ou son assistant, **caractérisé en ce que** le dispositif (10) est en outre conçu pour recueillir de la lumière dans un cône d'admission de lumière (47), au niveau d'une entrée de lumière (31), et la transférer à un dispositif d'analyse permettant d'analyser la lumière, sachant que la lumière est produite lors de l'utilisation de l'instrument chirurgical (11) et qu'une pointe ou une extrémité distale (50a) d'une électrode (15) ou de la partie de travail (15a) se situe à l'intérieur du cône d'admission de lumière (47) lorsque le dispositif (10) est fixé sur un instrument (11).

2. Dispositif (10) selon la revendication 1, dans lequel le dispositif (10) définit une sélection préétablie dans une seule ou dans plusieurs positions dans l'espace du dispositif de réception de lumière (24) fixé, par rapport au composant d'instrument (12) et/ou à l'instrument (11).

3. Dispositif (10) selon une des revendications précédentes, destiné à un instrument (11) qui présente une électrode (15) pouvant être alimentée en énergie électrique HF, le dispositif (10) définissant une sélection préétablie dans une seule ou dans plusieurs positions dans l'espace du dispositif de réception de lumière (24) fixé, par rapport à l'électrode (15).

4. Dispositif (10) selon une des revendications précédentes, le dispositif (10) portant une électrode (15) et définissant une sélection préétablie dans une seule ou dans plusieurs positions dans l'espace du dispositif de réception de lumière (24) par rapport à l'électrode (15).

5. Dispositif (10) selon une des revendications précédentes, comprenant un logement (25) qui limite la possibilité de mouvement de l'instrument dans le logement (25) à une possibilité de mouvement dans une direction axiale (L) et qui est conçu pour accueillir l'instrument (11) dans la direction axiale (L), et comprenant un dispositif (17, 51, 53, 21, 54, 55) destiné à réaliser ensuite la fixation ou la limitation de la position axiale de l'instrument (11) dans le logement (25).

6. Dispositif (10) selon la revendication 5, dans lequel le dispositif (17, 51, 53, 21, 54, 55) destiné à la fixation ou la limitation subséquente de la position axiale présente une partie (21, 22) qui peut être déplacée transversalement par rapport à la direction axiale (L) et est conçue pour être amenée en prise avec l'instrument (11), par déplacement latéral de la partie (21), après la mise en place de l'instrument (11) dans le logement (25), en vue de fixer ou limiter la position axiale de l'instrument (11) dans le logement (25).

7. Dispositif (10) selon la revendication 5, dans lequel la partie (21, 22) est fixée de manière souple.

8. Dispositif (10) selon une des revendications précédentes, dans lequel le dispositif (10) présente un canal de rinçage (28, 58), ledit canal de rinçage (28, 58) étant conçu pour délivrer un fluide de rinçage passant sur le côté de l'entrée de lumière (31) du dispositif de réception de lumière (24).

9. Dispositif (10) selon une des revendications précédentes, dans lequel le dispositif (10) définit la position dans l'espace du canal de rinçage par rapport au dispositif de réception de lumière (24).

10. Dispositif (10) selon une des revendications précédentes, dans lequel l'entrée de lumière (31) du dispositif de réception de lumière (24) est placée dans le dispositif (10).

11. Dispositif (10) selon une des revendications 8 à 10, dans lequel l'entrée de lumière (31) est disposée dans le canal de rinçage (58).

12. Dispositif (10) selon une des revendications précédentes, dans lequel le dispositif de réception de lumière (24) s'étend en totalité ou en partie dans le canal de rinçage (28).

13. Dispositif (10) selon une des revendications précédentes, dans lequel un tronçon de canal (58) est formé en étant délimité, sur un côté, par une paroi (45a, 45b, 46) du dispositif (10), et en étant délimité, sur l'autre côté, par un premier côté longitudinal (16a) d'une électrode (15), par exemple d'une tige d'électrode (15a), du dispositif (10), lequel tronçon de canal (58) peut être traversé par le fluide de rinçage, sachant qu'un deuxième côté longitudinal (16b) de l'électrode (15), par exemple un deuxième côté longitudinal (16b) de la tige d'électrode (15a), situé en vis-à-vis du premier côté longitudinal (16a), est dégagé au moins par portions le long du tronçon de canal (58).

14. Dispositif (10) selon une des revendications précédentes, dans lequel l'axe optique (OA) du dispositif de réception de lumière (24) et l'électrode (15) forment un angle aigu (W) entre eux.

15. Procédé de fixation d'un dispositif de réception de lumière (24) sur un instrument chirurgical (11) ou un composant d'instrument (12) de l'instrument chirurgical (11), au moyen d'un dispositif (10) selon une des revendications précédentes, par le chirurgien ou son assistant, pendant une opération.
